# EUROPEAN PATENT SPECIFICATION

(11) **EP 2 111 756 B1**
(45) Date of publication and mention of the grant of the patent: **02.08.2017**
(21) Application number: 09164961.6
(22) Date of filing: 31.05.2006
(51) Int. Cl.: A01P 7/04, A01N 43/90

(54) **PEST CONTROL AGENTS**
SCHÄDLINGSBEKÄMPFUNGSMITTEL
PRODUIT DE LUTTE CONTRE LES NUISIBLES

(30) Priority: 01.06.2005 JP 2005161019
(43) Date of publication of application: 28.10.2009
(62) Divisional of application: 06756816.2
(73) Proprietor: Meiji Seika Pharma Co., Ltd., Chuo-ku Tokyo (JP); The Kitasato Institute, Tokyo 108-8642 (JP)
(72) Inventor: Goto, Kimihiko, Yokohama-shi Kanagawa (JP); Horikoshi, Ryo, Yokohama-shi Kanagawa (JP); Tsuchida, Mariko, Yokohama-shi Kanagawa (JP); Oyama, Kazuhiko, Yokohama-shi Kanagawa (JP); Omura, Satoshi, Tokyo Tokyo (JP); Tomoda, Hiroshi, Tokyo Tokyo (JP); Sunazuka, Toshiaki, Funabashi-shi Chiba (JP)
(74) Representative: Regimbeau

(56) References cited:
- WO-A-94/09147
- WO-A-2004/060065
- JP-A- 8 259 569
- JP-A- 8 269 062
- JP-A- 8 269 065
- JP-A- 8 269 066
- WANG H-J ET AL: "Aflavinines and other antiinsectan metabolites from the ascostromata of Eupenicillium crustaceum and related species" APPLIED AND ENVIRONMENTAL MICROBIOLOGY, AMERICAN SOCIETY FOR MICROBIOLOGY, US, vol. 61, no. 12, 1 December 1995 (1995-12-01), pages 4429-4435, XP003023151 ISSN: 0099-2240
- OBATA R ET AL: "Chemical Modification and Structure-activity Relationships of Pyripyropenes. 3. Synthetic conversion of pyridine-pyrone moiety" JOURNAL OF ANTIBIOTICS, JAPAN ANTIBIOTICS RESEARCH ASSOCIATION, TOKYO, JP, vol. 50, no. 3, 1 March 1997 (1997-03-01), pages 229-236, XP003023150 ISSN: 0021-8820
- TOMODA H ET AL: "PYRIPYPROPENES, NOVEL INHIBITORS OF ACYL-COA:CHOLESTEROL ACYLTRANSFERASE PRODUCED BY ASPERGILLUS FUMIGATUS I. PRODUCTION, ISOLATION, AND BIOLOGICAL PROPERTIES" JOURNAL OF ANTIBIOTICS, JAPAN ANTIBIOTICS RESEARCH ASSOCIATION, TOKYO, JP, vol. 47, no. 2, 1 February 1994 (1994-02-01), pages 148-153, XP009060945 ISSN: 0021-8820
- OBATA R ET AL: "Chemical Modification and Structure-activity Relationships of Pyripyropenes. 1. Modification at the Four Hydroxyl Groups" JOURNAL OF ANTIBIOTICS, JAPAN ANTIBIOTICS RESEARCH ASSOCIATION, TOKYO, JP, vol. 49, no. 11, 1 January 1996 (1996-01-01), pages 1133-1148, XP003002774 ISSN: 0021-8820

## Description

### [BACKGROUND OF THE INVENTION]

### Field of Invention

The present invention relates to a composition for use as a pest control agent comprising a pyripyropene derivative as active ingredient.

### Background Art

Pyripyropene A has inhibitory activity against ACAT (acyl-CoA: cholesterol acyltransferase) and is expected to be applied, for example, to the treatment of diseases induced by cholesterol accumulation, as described in Japanese Patent No. 2993767 (Japanese Patent Laid-Open Publication No. 360895/1992) and Journal of Antibiotics (1993), 46(7), 1168-9.

Further, pyripyropene analogues and derivatives and ACAT inhibitory activity thereof are described in Journal of Society of Synthetic Organic Chemistry, Japan (1998), Vol. 56, No. 6, pp. 478-488, WO 94/09417, Japanese Patent Laid-Open Publication No. 184158/1994, Japanese Patent Laid-Open Publication No. 239385/1996, Japanese Patent Laid-Open Publication No. 259569/1996, Japanese Patent Laid-Open Publication No. 269062/1996, Japanese Patent Laid-Open Publication No. 269063/1996, Japanese Patent Laid-Open Publication No. 269064/1996, Japanese Patent Laid-Open Publication No. 269065/1996, Japanese Patent Laid-Open Publication No. 269066/1996, Japanese Patent Laid-Open Publication No. 291164/1996, and Journal of Antibiotics (1997), 50(3), 229-36.

Furthermore, Applied and Environmental Microbiology (1995), 61(12), 4429-35 describes that pyripyropene A has insecticidal activity against larvae of Helicoverpa zea. Furthermore, WO 2004/060065 describes that pyripyropene A has insecticidal activity against Plutella xylostella L larvae and Tenebrio molitor L. In these documents, however, there is no specific description on insecticidal activity of pyripyropene A against other pests.

Further, none of the above documents describes insecticidal activity of pyripyropene analogues and derivatives.

Up to now, many compounds having insecticidal activity have been reported and have been used as pest control agents. However, the presence of insect species, which are resistant to or can be hardly controlled by these compounds, has posed a problem. Accordingly, the development of a novel pest control agent having excellent insectidal activity has still been desired.

### [SUMMARY OF THE INVENTION]

The present inventors have now found that pyripyropene derivatives represented by formula (I) have significant insecticidal activity.

The present inventors further found that pyripyropene A and its derivatives represented by formula (Ia) have significant insecticidal activity against hemipteran pests.

Furthermore, the present inventors have found novel pyripyropene derivatives represented by formula (Ib) having significant insecticidal activity.

The present invention has been made based on such finding.

Accordingly, an object of the present invention is to provide a composition useful as a pest control agent, that comprises a pyripyropene derivative having significant insecticidal activity as active ingredient and can reliably exhibit the contemplated effect and can be used safely. Another object of the present invention is to provide a hemipteran pest control agent that comprises pyripyropene A and its derivative as active ingredient and can reliably exhibit the contemplated effect and can be used safely. A further object of the present invention is to provide a novel pyripyropene derivative having significant insecticidal activity.

There is described a composition for use as a pest control agent, comprising a compound represented by formula (I) or an agriculturally and horticulturally acceptable salt thereof as active ingredient and an agriculturally and horticulturally acceptable carrier: 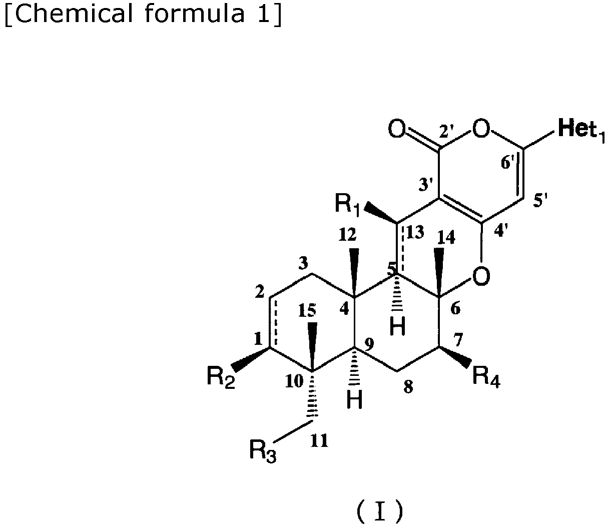 wherein
Het₁ represents optionally substituted 3-pyridyl,
R₁ represents hydroxyl,
   optionally substituted C₁₋₆ alkylcarbonyloxy,
   optionally substituted C₂₋₆ alkenylcarbonyloxy,
   optionally substituted C₂₋₆ alkynylcarbonyloxy,
   optionally substituted C₁₋₆ alkyloxy,
   optionally substituted C₂₋₆ alkenyloxy,
   optionally substituted C₂₋₆ alkynyloxy,
   optionally substituted benzyloxy, or
   oxo in the absence of a hydrogen atom at the 13-position, or
the bond between 5-position and 13-position represents a double bond in the absence of R₁ and a hydrogen atom at the 5-position,
   R₂ represents hydroxyl,
   optionally substituted C₁₋₁₈ alkylcarbonyloxy,
   optionally substituted C₂₋₆ alkenylcarbonyloxy,
   optionally substituted C₂₋₆ alkynylcarbonyloxy,
   optionally substituted benzoyloxy, or
   optionally substituted C₁₋₆ alkylsulfonyloxy,
   R₃ represents a hydrogen atom,
   hydroxyl,
   optionally substituted C₁₋₁₈ alkylcarbonyloxy,
   optionally substituted C₂₋₆ alkenylcarbonyloxy,
   optionally substituted C₂₋₆ alkynylcarbonyloxy,
   optionally substituted benzoyloxy,
   optionally substituted C₁₋₆ alkylsulfonyloxy,
   optionally substituted benzenesulfonyloxy, or
   optionally substituted five- or six-membered heterocyclic thiocarbonyloxy, or
R₂ and R₃ together represent -O-CR₂'R₃'-O- wherein R₂' and R₃', which may be the same or different, represent a hydrogen atom, C₁₋₆ alkyl, C₁₋₆ alkyloxy, C₂₋₆ alkenyl, optionally substituted phenyl, or optionally substituted benzyl, or R₂' and R₃' together represent oxo or C₂₋₆ alkylene, and
R₄ represents a hydrogen atom,
   hydroxyl,
   optionally substituted C₁₋₁₈ alkylcarbonyloxy,
   optionally substituted C₂₋₆ alkenylcarbonyloxy,
   optionally substituted C₂₋₆ alkynylcarbonyloxy,
   optionally substituted benzoyloxy,
   optionally substituted C₁₋₆ alkylsulfonyloxy,
   optionally substituted benzenesulfonyloxy,
   optionally substituted benzyloxy,
   optionally substituted C₁₋₆ alkyloxy,
   optionally substituted C₂₋₆ alkenyloxy,
   optionally substituted C₂₋₆ alkynyloxy,
   C₁₋₆ alkyloxy-C₁₋₆ alkyloxy,
   C₁₋₆ alkylthio-C₁₋₆ alkyloxy,
   C₁₋₆ alkyloxy-C₁₋₆ alkyloxy-C₁₋₆ alkyloxy,
   optionally substituted C₁₋₆ alkyloxycarbonyloxy,
   optionally substituted C₁₋₆ alkylaminocarbonyloxy,
   optionally substituted saturated or unsaturated
   five- or six-membered heterocyclic oxy,
   optionally substituted saturated or unsaturated five- or six-membered heterocyclic carbonyloxy,
   optionally substituted thieno[3,2-b]pyridylcarbonyloxy,
   optionally substituted 1H-indolylcarbonyloxy,
   optionally substituted saturated or unsaturated five- or six-membered heterocyclic thiocarbonyloxy, or
   oxo in the absence of a hydrogen atom at the 7-position, provided that
   a compound wherein
   Het₁ represents 3-pyridyl,
   R₁ represents hydroxyl, and
   all of R₂, R₃, and R₄ represent acetyloxy,
   is excluded.

According to one aspect of the present invention, there is provided a non therapeutic use of a compound represented by formula (Ia) or an agriculturally and horticulturally acceptable salt thereof as hemipteran pest control agent, and a method for controlling a hemipteran pest, comprising applying an effective amount of a compound represented by formula (Ia), or an agriculturally and horticulturally acceptable salt thereof to a plant or soil: 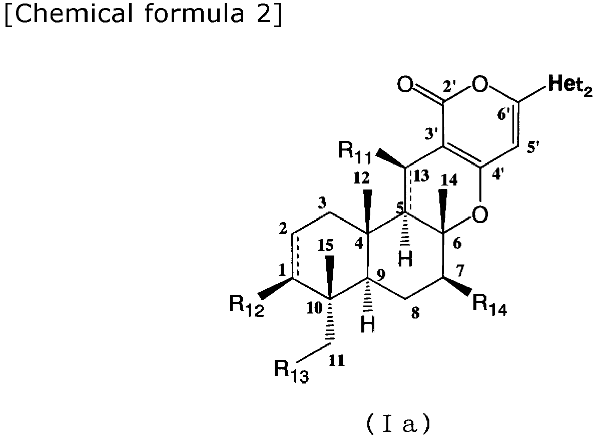 wherein
Het₂ represents 3-pyridyl,
R₁₁ represents hydroxyl,
R₁₂ represents acetoxy,
R₁₃ represents acetoxy,
R₁₄ represents acetoxy

Further, the pyripyropene derivative according to the present invention comprises a compound represented by formula (Ib) or an agriculturally and horticulturally acceptable salt thereof: 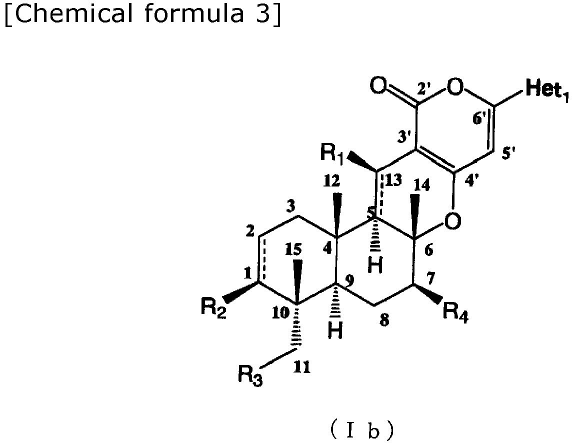 wherein
Het₁ represents 3-pyridyl,
R₁ represents hydroxyl,
R₂ and R₃ represent propionyloxy or optionally substituted cyclic C₃₋₆ alkylcarbonyloxy, and
R₄ represents hydroxyl,
   optionally substituted cyclic C₃₋₆ alkylcarbonyloxy,
   optionally substituted benzoyloxy, or
   optionally substituted saturated or unsaturated five- or six-membered heterocyclic carbonyloxy.

The pyripyropene derivatives represented by formula (Ia) or formula (Ib) according to the present invention have excellent control effect against agricultural and horiticultural pests, sanitary pests, parasites of animals, stored grain pests, clothing pests, and house pests and a compositions comprising the pyripyropene derivatives as active ingredient can be advantageously utilized as a novel pest control agent. Therefore, in another aspect, the present invention concerns a composition for use as a pest control agent comprising the compound of formula (Ib), or an agriculturally and horticulturally acceptable salt thereof, as active ingredient and an agriculturally and horticulturally acceptable carrier. In another aspect, the present invention concerns a non-therapeutic use of a compound represented by formula (Ib), or an agriculturally and horticulturally acceptable salt thereof, as a pest control agent. According to another aspect, the present invention concerns a method for controlling an pest, comprising applying an effective amount of a compound represented by formula (Ib), or an agriculturally and horticulturally acceptable salt thereof to a plant or soil.

Further, it is surprising that, among the compounds represented by formula (Ia), pyripyropene A has excellent control effect against hemipteran pests. Accordingly, a composition according to the present invention comprising the compounds represented by formula (Ia) including pyripyropene A, can be advantageously utilized particularly as hemipteran pest control agent.

### [DETAILED DESCRIPTION OF THE INVENTION]

The term "halogen" as used herein means fluorine, chlorine, bromine, or iodine, preferably fluorine, chlorine, or bromine.

The terms "alkyl," "alkenyl," and "alkynyl" as used herein as a group or a part of a group respectively mean alkyl, alkenyl, and alkynyl that the group is of a straight chain, branched chain, or cyclic type or a type of a combination thereof unless otherwise specified. Further, for example, "C₁₋₆" in "C₁₋₆ alkyl" as used herein as a group or a part of a group means that the number of carbon atoms in the alkyl group is 1 to 6. Further, in the case of cyclic alkyl, the number of carbon atoms is at least three.

The term "heterocyclic ring" as used herein means a heterocyclic ring containing one or more, preferably one to four, heteroatoms, which may be the same or different, selected from the group consisting of nitrogen, oxygen, and sulfur atoms. Further, the expression "optionally substituted" alkyl as used herein means that one or more hydrogen atoms on the alkyl group may be substituted by one or more substituents which may be the same or different. It will be apparent to a person having ordinary skill in the art that the maximum number of substituents may be determined depending upon the number of substitutable hydrogen atoms on the alkyl group. This is true of functional groups other than the alkyl group.

3-Pyridyl represented by Het₁ and Het₂ is optionally substituted, and substituents include halogen atoms, C₁₋₄ alkyl, C₁₋₄ alkyloxy, nitro, cyano, formyl, trifluoromethyl, trifluoromethoxy, trifluoromethylthio, trifluoromethylsulfinyl, trifluoromethylsulfonyl, acetyl, and acetyloxy. Preferred are halogen atoms and trifluoromethyl. A chlorine atom and trifluoromethyl are more preferred.

"C₁₋₁₈ alkylcarbonyloxy" represented by R₂, R₃ and R₄, and R₁₂, R₁₃ and R₁₄ is preferably C₁₋₆ alkylcarbonyloxy, more preferably propionyloxy or cyclic C₃₋₆ alkylcarbonyloxy. The C₁₋₁₈ alkylcarbonyloxy group is optionally substituted, and substituents include halogen atoms, cyano, cyclic C₃₋₆alkyl, phenyl, trifluoromethoxy, trifluoromethylthio, pyridyl, and pyridylthio. More preferred are halogen atoms, cyclic C₃₋₆ alkyl, and pyridyl.

"C₁₋₆ alkyloxy" represented by R₁ and R₄, and R₁₁ and R₁₄ is optionally substituted, and substituents include halogen atoms; cyano; phenyl; trifluoromethoxy; trifluoromethylthio; C₁₋₆ alkylcarbonyl optionally substituted by a halogen atom; and C₁₋₆ alkylcarbonyloxy optionally substituted by a halogen atom.

Phenyl in "benzyloxy" represented by R₄, and R₁₄ is optionally substituted, and substituents include halogen atoms; C₁₋₆ alkyloxy optionally substituted by a halogen atom.

Phenyl in "benzoyloxy" represented by R₄ is optionally substituted, and substituents include halogen atoms; C₁₋₆ alkyloxy optionally substituted by a halogen atom; C₁₋₆ alkyl optionally substituted by a halogen atom; C₁₋₆ alkylcarbonyl optionally substituted by a halogen atom; C₁₋₆ alkylcarbonyloxy optionally substituted by a halogen atom; C₁₋₆ alkylcarbonylamino optionally substituted by a halogen atom; C₁₋₆ alkylaminocarbonyloxy optionally substituted by a halogen atom; C₁₋₆ alkylaminocarbonyl optionally substituted by a halogen atom; C₁₋₆ alkylsulfonyloxy optionally substituted by a halogen atom; C₁₋₆ alkylthio optionally substituted by a halogen atom; C₁₋₆ alkylsulfinyl optionally substituted by a halogen atom; C₁₋₆ alkylsulfonyl optionally substituted by a halogen atom; cyano; nitro; formyl; azide; guanidyl; group -C(=NH)-NH₂; and group -CH=N-O-CH₃. Preferred are halogen atoms, C₁₋₆ alkyl substituted by a halogen atom, cyano, and nitro.

"Saturated or unsaturated five- or six-membered heterocyclic ring" in "saturated or unsaturated five- or six-membered heterocyclic oxy," "saturated or unsaturated five-or six-membered heterocyclic carbonyloxy," and "saturated or unsaturated five- or six-membered heterocyclic thiocarbonyloxy" represented by R₄ is saturated or unsaturated five- or six-membered heterocyclic ring containing one to three heteroatoms selected from the group consisting of nitrogen, oxygen, and sulfur atoms, more preferably, saturated or unsaturated five- or six-membered heterocyclic ring containing one or two heteroatoms selected from the group consisting of nitrogen, oxygen, and sulfur atoms, more preferably, saturated or unsaturated five- or six-membered heterocyclic ring containing one or two nitrogen atoms, saturated or unsaturated five- or six-membered heterocyclic ring containing one or two oxygen atoms, saturated or unsaturated five- or six-membered heterocyclic ring containing one or two sulfur atoms, saturated or unsaturated five- or six-membered heterocyclic ring containing one nitrogen atom and one oxigen atom, or saturated or unsaturated five- or six-membered heterocyclic ring containing one nitrogen atom and one sulfur atom.

In the present invention, the "saturated or unsaturated five- or six-membered heterocyclic ring" are thienyl, furyl, pyrrolyl, imidazolyl, pyrazolyl, isothiazoyl, isoxazolyl, thiazolyl, oxazolyl, pyridyl, pyrimidinyl, pyrazinyl, pyridazinyl, tetrahydropyranyl, piperidinyl, piperazinyl, morpholinyl, and mannosyl. Preferred are pyridyl, furanyl, thiazolyl, imidazolyl, tetrahydropyranyl, and mannosyl. More speciific examples thereof include (2- or 3-)thienyl, (2- or 3-)furyl, (1-, 2- or 3-)pyrrolyl, (1-, 2-, 4- or 5-)imidazolyl, (1-, 3-, 4- or 5-)pyrazolyl, (3-, 4- or 5-)isothiazoyl, (3-, 4- or 5-)isoxazolyl, (2-, 4- or 5-)thiazolyl, (2-, 4- or 5-)oxazolyl, (2-, 3- or 4-)pyridyl or , (2-, 4-, 5- or 6-)pyrimidinyl, (2- or 3-)pyrazinyl, (3- or 4-)pyridazinyl, (2-, 3- or 4-)tetrahydropyranyl, (1-, 2-, 3- or 4-)piperidinyl, (1-, 2- or 3-)piperazinyl, and (2-, 3- or 4-)morpholinyl, preferably 3-pyridyl, 2-franyl, 5-thiazolyl, 1-imidazolyl, 5-imidazolyl, and 2-tetrahydropyranyl, more preferably 2-tetrahydropyranyl, 2-pyrazinyl, and 3-pyridyl, particularly preferably 3-pyridyl.

The heterocyclic ring in the "saturated or unsaturated five- or six-membered heterocyclic carbonyloxy" and "saturated or unsaturated five- or six-membered heterocyclic thiocarbonyloxy" and "thieno[3,2-b]pyridylcarbonyloxy" and "1H-indolylcarbonyloxy" represented by R₄ are optionally substituted, and substituents include halogen atoms, C₁₋₄ alkyl, C₁₋₄ alkyloxy, C₁₋₄ alkylthio, nitro, cyano, formyl, trifluoromethoxy, trifluoromethyl, trifluoromethylthio, trifluoromethylsulfinyl, trifluoromethylsulfonyl, acetyl, acetyloxy, benzoyl, and C₁₋₄ alkyloxycarbonyl. Preferred are halogen atoms, C₁₋₄ alkyl, C₁₋₄ alkyloxy, and trifluoromethyl.

### A composition for use as a hemipteran pest control agent, comprising a compound represented by formula (Ia)

According to the present invention, in the compound represented by formula (Ia), Het₂ represents 3-pyridyl.

Further, according to the present invention, in the compound represented by formula (Ia), R₁₁ represents hydroxyl.

According to the present invention, in the compound represented by formula (Ia), R₁₂ represents acetyloxy [0046] In the present invention, in the compound represented by formula (Ia), R₁₃ represents acetyloxy.

According to the present invention, in the compound represented by formula (Ia), R₁₄ represents acetyloxy.

Further, an agriculturally and horticulturally acceptable salt of the compound represented by formula (Ia) include the same as that of the compound represented by formula (Ib) described below.

### Compunds of formula (Ib) or its agriculturally and horticulturally acceptable salts

Compounds of formula (Ib) are novel pyripyropene derivatives that are comprised as a part in the compound represented by formula (I). In particular, they have significant insecticidal activity.

According to an embodiment of the present invention, there is provided the compounds of formula (Ib), excluding a compound wherein Het₁ represents 3-pyridyl, R₁ represents hydroxyl, and R₂ and R₃ represent propionyloxy, and R₄ represents hydroxyl.

According to another preferred embodiment of the present invention, in the compound represented by formula (Ib), R₂ and R₃ represent optionally substituted cyclic C₃₋₆ alkylcarbonyloxy, R₄ represents hydroxyl, optionally substituted cyclic C₃₋₆ alkylcarbonyloxy, or optionally substituted benzoyloxy. Alternatively, R₂ and R₃ represent propionyloxy, R₄ represents optionally substituted cyclic C₃₋₆ alkylcarbonyloxy, or optionally substituted saturated or unsaturated five- or six-membered heterocyclic carbonyloxy.

According to another preferred embodiment of the present invention, in the compounds represented by formula (Ib), R₂ and R₃ represent optionally substituted cyclic C₃₋₆ alkylcarbonyloxy, R₄ represents hydroxyl, optionally substituted cyclic C₃₋₆ alkylcarbonyloxy, or optionally substituted benzoyloxy.

According to another preferred embodiment of the present invention, in the compounds represented by formula (Ib), R₂ and R₃ represent propionyloxy, R₄ represents optionally substituted cyclic C₃₋₆ alkylcarbonyloxy or optionally substituted saturated or unsaturated five- or six-membered heterocyclic carbonyloxy.

According to still another preferred embodiment of the present invention, there is provided a composition for use as a pest control agent comprising a compound represented by formula (Ib) or an agriculturally and horticulturally acceptable salt thereof as an active ingredient and an agriculturally and horticulturally acceptable carrier.

Agriculturally and horticulturally acceptable salts in the compounds of formula (Ib) include, for example, acid addition salts such as hydrochlorides, nitrates, sulfates, phosphates, or acetates.

Specific examples of the compounds represented by formula (Ia), or (Ib) include certain compounds shown in Tables 1 to 14 below. In the following tables, H(=) means that the bond between 5-position and 13-position represents a double bond in the absence of R₁ and a hydrogen atom at the 5-position .
[Table 1]

**Table 1**

| Reference Compound No. | R₁ | R₂ | R₃ | R₄ | Het₁ |
|---|---|---|---|---|---|
| 1 | OH | OCOCH₃ | OCOCH₃ | OCOCH₂CH₃ | 3-pyridyl |
| 2 | OH | OCOCH₃ | OCOCH₃ | OCOCH₂CF₃ | 3-pyridyl |
| 3 | OH | OCOCH₃ | OCOCH₃ | OCOCH₂OCH₃ | 3-pyridyl |
| 4 | OH | OCOCH₃ | OCOCH₃ | OCOCH₂OCOCH₃ | 3-pyridyl |
| 5 | OH | OCOCH₃ | OCOCH₃ | OCOCH₂CH₂CN | 3-pyridyl |
| 6 | OH | OCOCH₃ | OCOCH₃ | OCO(CH₂)₂CH₃ | 3-pyridyl |
| 7 | OH | OCOCH₃ | OCOCH₃ | OCO(CH₂)₃CH₃ | 3-pyridyl |
| 8 | OH | OCOCH₃ | OCOCH₃ | OCO(CH₂)₄CH₃ | 3-pyridyl |
| 9 | OH | OCOCH₃ | OCOCH₃ | OCO(CH₂)₅CH₃ | 3-pyridyl |
| 10 | OH | OCOCH₃ | OCOCH₃ | OCO(CH₂)₆CH₃ | 3-pyridyl |
| 11 | OH | OCOCH₃ | OCOCH₃ | OCO(CH₂)₁₆CH₃ | 3-pyridyl |
| 12 | OH | OCOCH₃ | OCOCH₃ | OCOCH(CH₃)₂ | 3-pyridyl |
| 13 | OH | OCOCH₃ | OCOCH₃ | OCOC(CH₃)₃ | 3-pyridyl |
| 14 | OH | OCOCH₃ | OCOCH₃ | OCOCH₂CH(CH₃)₂ | 3-pyridyl |
| 15 | OH | OCOCH₃ | OCOCH₃ | OCO(CH₂)₂CH(CH₃)₂ | 3-pyridyl |
| 16 | OH | OCOCH₃ | OCOCH₃ | OCO-trans-CH=CHCH₂CH₃ | 3-pyridyl |
| 17 | OH | OCOCH₃ | OCOCH₃ | OCOCH₂C≡CCH₃ | 3-pyridyl |
| 18 | OH | OCOCH₃ | OCOCH₃ | OCOC≡CCH₂CH₃ | 3-pyridyl |
| 19 | OH | OCOCH₃ | OCOCH₃ | OCO(CH₂)₂C≡CH | 3-pyridyl |
| 20 | OH | OCOCH₃ | OCOCH₃ | OCO(CH₂)₂CH=CH₂ | 3-pyridyl |

[Table 2]

**Table 2**

| Reference Compound No. | R₁ | R₂ | R₃ | R₄ | Het₁ |
|---|---|---|---|---|---|
| 21 | OH | OCOCH₃ | OCOCH₃ | OCOCH₂C₆H₅ | 3-pyridyl |
| 22 | OH | OCOCH₃ | OCOCH₃ | OCO(CH₂)₂C₆H₅ | 3-pyridyl |
| 23 | OH | OCOCH₃ | OCOCH₃ | OCOC₆H₅ | 3-pyridyl |
| 24 | OH | OCOCH₃ | OCOCH₃ | OCO-(4-Br-C₆H₄) | 3-pyridyl |
| 25 | OH | OCOCH₃ | OCOCH₃ | OCO-(4-N₃-C₆H₄) | 3-pyridyl |
| 26 | OH | OCOCH₃ | OCOCH₃ | OCO-(4-OCF₃-C₆H₄) | 3-pyridyl |
| 27 | OH | OCOCH₃ | OCOCH₃ | OCO-(4-SO₂CF₃-C₆H₄) | 3-pyridyl |
| 28 | OH | OCOCH₃ | OCOCH₃ | OCO-(3-pyridyl) | 3-pyridyl |
| 29 | OH | OCOCH₃ | OCOCH₃ | OCO-(2-Cl-3-pyridyl) | 3-pyridyl |
| 30 | OH | OCOCH₃ | OCOCH₃ | OCO-(2-franyl) | 3-pyridyl |
| 31 | OH | OCOCH₃ | OCOCH₃ | OCO-(2-thiazolyl) | 3-pyridyl |
| 32 | OH | OCOCH₃ | OCOCH₃ | OCO-(2-Cl-5-thiazolyl) | 3-pyridyl |
| 33 | OH | OCOCH₃ | OCOCH₃ | OCO-(5-imidazolyl) | 3-pyridyl |
| 34 | OH | OCOCH₃ | OCOCH₃ | OCS-(1-imidazolyl) | 3-pyridyl |
| 35 | OH | OCOCH₃ | OCOCH₃ | OCOOCH₂C₆H₅ | 3-pyridyl |
| 36 | OH | OCOCH₃ | OCOCH₃ | OSO₂CH₃ | 3-pyridyl |
| 37 | OH | OCOCH₃ | OCOCH₃ | OSO₂C₆H₅ | 3-pyridyl |
| 38 | OH | OCOCH₃ | OCOCH₃ | OCONHCH₂CH₃ | 3-pyridyl |
| 39 | OH | OCOCH₃ | OCOCH₃ | OCONH(CH₂)₂CH₃ | 3-pyridyl |
| 40 | OH | OCOCH₃ | OCOCH₃ | OCONHCH₂C₆H₅ | 3-pyridyl |

[Table 3]

**Table 3**

| Reference Compound No. | R₁ | R₂ | R₃ | R₄ | Het₁ |
|---|---|---|---|---|---|
| 41 | OH | OCOCH₃ | OCOCH₃ | OCH₂C₆H₅ | 3-pyridyl |
| 42 | OH | OCOCH₃ | OCOCH₃ | OCH₂SCH₃ | 3-pyridyl |
| 43 | OH | OCOCH₃ | OCOCH₃ | OCH₂OCH₃ | 3-pyridyl |
| 44 | OH | OCOCH₃ | OCOCH₃ | OCH₂OCH₂CH₂OCH₃ | 3-pyridyl |
| 45 | OH | OCOCH₃ | OCOCH₃ | O-(2-tetrahydropyranyl) | 3-pyridyl |
| 46 | OH | OCOCH₃ | OCOCH₃ | O-(tetra-O-benzyl-mannosyl) | 3-pyridyl |
| 47 | OH | OCOCH₃ | OCOCH₃ | H | 3-pyridyl |
| 48 | OH | OCOCH₃ | OCOCH₃ | OCO-c-C₃H₅ | 3-pyridyl |
| 49 | OH | OCOCH₃ | OCOCH₃ | OH | 3-pyridyl |
| 50 | OH | OCOCH₃ | OCOCH₃ | =O | 3-pyridyl |
| 51 | OH | OCOCH₃ | OCOCH2CH3 | OCOCH₃ | 3-pyridyl |
| 52 | OH | OCOCH₃ | OCOCH2CH3 | OCOCH₂CH₃ | 3-pyridyl |
| 53 | OH | OCOCH₃ | OCOCH₂CH₃ | H | 3-pyridyl |
| 54 | OH | OCOCH₃ | OCO(CH₂)₂CH₃ | OCOCH₃ | 3-pyridyl |
| 55 | OH | OCOCH₃ | OCO(CH₂)₂CH₃ | OH | 3-pyridyl |
| 56 | OH | OCOCH₃ | OCO(CH₂)₃CH₃ | OCOCH₃ | 3-pyridyl |
| 57 | OH | OCOCH₃ | OCOCH(CH₃)₂ | OCOCH₃ | 3-pyridyl |
| 58 | OH | OCOCH₃ | OCOC₆H₅ | OCOCH₃ | 3-pyridyl |
| 59 | OH | OCOCH₃ | OCOC₆H₅ | OH | 3-pyridyl |
| 60 | OH | OCOCH₃ | OCS-(1-imidazolyl) | OCOCH₃ | 3-pyridyl |

[Table 4]

**Table 4**

| Compound No. | R₁ | R₂ | R₃ | R₄ | Het₁ |
|---|---|---|---|---|---|
| Reference compound 61 | OH | OCOCH₃ | OSO₂CH₃ | OCOCH₃ | 3-pyridyl |
| Reference compound 62 | OH | OCOCH₃ | OSO₂CH₃ | OCO(CH₂)₃CH₃ | 3-pyridyl |
| Reference compound 63 | OH | OCOCH₃ | OSO₂C₆H₅ | OCOCH₃ | 3-pyridyl |
| Reference compound 64 | OH | OCOCH₃ | OSO₂CH₂CH₃ | OCOCH₃ | 3-pyridyl |
| Reference compound 65 | OH | OCOCH₃ | OSO₂CH₂CH₂CH₃ | OCOCH₃ | 3-pyridyl |
| Reference compound 66 | OH | OCOCH₃ | OSO₂CH₂CH₃ | OH | 3-pyridyl |
| Reference compound 67 | OH | OCOCH₃ | OSO₂CH₂CH₂CH₃ | OH | 3-pyridyl |
| Reference compound 68 | OH | OCOCH₃ | OH | OH | 3-pyridyl |
| Reference compound 69 | OH | OCOCH₃ | OH | OCOCH₃ | 3-pyridyl |
| Reference compound 70 | OH | OCOCH₃ | H | H | 3-pyridyl |
| Reference compound 71 | OH | OCOCH₃ | H | OCOCH₂CH₃ | 3-pyridyl |
| Reference compound 72 | OH | OCOCH₂CH₃ | OCOCH₃ | OCOCH₃ | 3-pyridyl |
| 73 | OH | OCOCH₂CH₃ | OCOCH₂CH₃ | OH | 3-pyridyl |
| 74 | OH | OCOCH₂CH₃ | OCOCH₂CH₃ | OCOCH₃ | 3-pyridyl |
| Reference compound 75 | OH | OCOCH₂CH₃ | OCOCH₃ | OCOCH₂CH₃ | 3-pyridyl |
| Reference compound 76 | OH | OCOCH₂CH₃ | OCOCH₂CH₃ | OCOCH₂CH₃ | 3-pyridyl |
| Reference compound 77 | OH | OCOCH₂CH₃ | OCOCH₂CH₃ | OCOC₆H₅ | 3-pyridyl |
| Reference compound 78 | OH | OCOCH₂CH₃ | OCOCH₂CH₃ | H | 3-pyridyl |
| Reference compound 79 | OH | OCOCH₂CH₃ | H | H | 3-pyridyl |
| Reference compound 80 | OH | OCO(CH₂)₂CH₃ | OCOCH₃ | OCOCH₃ | 3-pyridyl |

[Table 5]

**Table 5**

| Reference Compound No. | R₁ | R₂ | R₃ | R₄ | Het₁ |
|---|---|---|---|---|---|
| 81 | OH | OCO(CH₂)₂CH₃ | OCO(CH₂)₂CH₃ | OH | 3-pyridyl |
| 82 | OH | OCO(CH₂)₂CH₃ | OCO(CH₂)₂CH₃ | OCO(CH₂)₂CH₃ | 3-pyridyl |
| 83 | OH | OCO(CH₂)₂CH₃ | OCO(CH₂)₂CH₃ | OCOCH₃ | 3-pyridyl |
| 84 | OH | OCO(CH₂)₃CH₃ | OCOCH₃ | OCOCH₃ | 3-pyridyl |
| 85 | OH | OCO(CH₂)₃CH₃ | OCO(CH₂)₃CH₃ | OCO(CH₂)₃CH₃ | 3-pyridyl |
| 86 | OH | OCO(CH₂)₃CH₃ | OSO₂CH₃ | OCO(CH₂)₃CH₃ | 3-pyridyl |
| 87 | OH | OCO(CH₂)₃CH₃ | OSO₂CH₃ | OH | 3-pyridyl |
| 88 | OH | OCO(CH₂)₁₆CH₃ | OCO(CH₂)₁₆CH₃ | OCO(CH₂)₁₆CH₃ | 3-pyridyl |
| 89 | OH | OCOCH(CH₃)₂ | OCOCH₃ | OCOCH₃ | 3-pyridyl |
| 90 | OH | OCOCH(CH₃)₂ | OCOCH(CH₃)₂ | OCOCH(CH₃)₂ | 3-pyridyl |
| 91 | OH | OCOC(CH₃)₃ | OCOC(CH₃)₃ | OCOC(CH₃)₃ | 3-pyridyl |
| 92 | OH | OCOC₆H₅ | OCOCH₃ | OCOCH₃ | 3-pyridyl |
| 93 | OH | OCOC₆H₅ | OSO₂CH₃ | OH | 3-pyridyl |
| 94 | OH | OCOC₆H₅ | OSO₂CH₃ | OCOCH₃ | 3-pyridyl |
| 95 | OH | OCOC₆H₅ | OSO₂CH₃ | OCO(CH₂)₃CH₃ | 3-pyridyl |
| 96 | OH | OCO-(4-Br-C₆H₄) | OCO-(4-Br-C₆H₄) | OCO-(4-Br-C₆H₄) | 3-pyridyl |
| 97 | OH | OCO-(4-N₃-C₆H₄) | OSO₂CH₃ | OCOCH₃ | 3-pyridyl |
| 98 | OH | OSO₂CH₃ | OSO₂CH₃ | OH | 3-pyridyl |
| 99 | OH | OSO₂CH₃ | OSO₂CH₃ | OSO₂CH₃ | 3-pyridyl |
| 100 | OH | OSO₂CH₃ | OSO₂CH₃ | OCOCH₃ | 3-pyridyl |

[Table 6]

**Table 6**

| Reference Compound No. | R₁ | R₂ | R₃ | R₄ | Het₁ |
|---|---|---|---|---|---|
| 101 | OH | OSO₂CH₃ | OH | OH | 3-pyridyl |
| 102 | OH | OH | OH | OH | 3-pyridyl |
| 103 | OH | OH | OH | OCOCH₃ | 3-pyridyl |
| 104 | OH | OH | OH | OCO(CH₂)₃CH₃ | 3-pyridyl |
| 105 | OH | OH | OH | OCH₂OCH₂CH₂OCH₃ | 3-pyridyl |
| 106 | OH | OH | OCOCH₃ | OH | 3-pyridyl |
| 107 | OH | OH | OCOCH₂CH₃ | OH | 3-pyridyl |
| 108 | OH | OH | OCO(CH₂)₂CH₃ | OH | 3-pyridyl |
| 109 | OH | OH | OCO(CH₂)₃CH₃ | OH | 3-pyridyl |
| 110 | OH | OH | OCOCH(CH₃)₂ | OH | 3-pyridyl |
| 111 | OH | OH | OSO₂CH₃ | OH | 3-pyridyl |
| 112 | OH | OH | OSO₂CH₂CH₃ | OH | 3-pyridyl |
| 113 | OH | OH | OSO₂CH₂CH₂CH₃ | OH | 3-pyridyl |
| 114 | OH | OH | OSO₂CH(CH₃)₂ | OH | 3-pyridyl |
| 115 | OH | OH | OSO₂C₆H₅ | OH | 3-pyridyl |
| 116 | OH | OH | OSO₂-(4-CH₃-C₆H₄) | OH | 3-pyridyl |
| 117 | OH | OH | OCO-(4-Br-C₆H₄) | OH | 3-pyridyl |
| 118 | OH | OH | OCO(CH₂)₃CH₃ | OCO(CH₂)₃CH₃ | 3-pyridyl |
| 119 | OH | OH | OSO₂CH₃ | OSO₂CH₃ | 3-pyridyl |
| 120 | OH | OH | OSO₂CH₃ | OCOCH₃ | 3-pyridyl |

[Table 7]

**Table 7**

| Reference Compound No. | R₁ | R₂ | R₃ | R₄ | Het₁ |
|---|---|---|---|---|---|
| 121 | OH | OH | OSO₂CH₃ | OCOCH₃ | 3-pyridyl |
| 122 | OH | OH | OSO₂CH₃ | OCO(CH₂)₃CH₃ | 3-pyridyl |
| 123 | OH | OH | OSO₂C₆H₅ | OCOCH₃ | 3-pyridyl |
| 124 | OH | OH | OSO₂C₆H₅ | OSO₂C₆H₅ | 3-pyridyl |
| 125 | OH | -O-CH(CH₃)-O- | | OCO(CH₂)₃CH₃ | 3-pyridyl |
| 126 | OH | -O-CH(C₂H₅)-O- | | OH | 3-pyridyl |
| 127 | OH | -O-CH(C₂H₅)-O- | | OCO(CH₂)₃CH₃ | 3-pyridyl |
| 128 | OH | -O-CH(CH=CH₂)-O- | | OH | 3-pyridyl |
| 129 | OH | -O-CH(CH=CH₂)-O- | | OCO(CH₂)₃CH₃ | 3-pyridyl |
| 130 | OH | -O-CH(CH(CH₃)₂)-O- | | OH | 3-pyridyl |
| 131 | OH | -O-CH(CH(CH₃)₂)-O- | | OCO(CH₂)₃CH₃ | 3-pyridyl |
| 132 | OH | -O-CH(OCH₃)-O- | | OH | 3-pyridyl |
| 133 | OH | -O-CH(C(CH₃)₃)-O- | | OCO(CH₂)₃CH₃ | 3-pyridyl |
| 134 | OH | -O-CH(CH₂C₆H₅)-O- | | OH | 3-pyridyl |
| 135 | OH | -O-C(CH₃)₂-O- | | OH | 3-pyridyl |
| 136 | OH | -O-C(CH₃)₂-O- | | OCOCH₃ | 3-pyridyl |
| 137 | OH | -O-C(CH₃)₂-O- | | OCO(CH₂)₃CH₃ | 3-pyridyl |
| 138 | OH | -O-C(CH₃)(C₆H₅)-O- | | OH | 3-pyridyl |
| 139 | OH | -O-C(CH₃)(C₆H₅)-O- | | OCO(CH₂)₃CH₃ | 3-pyridyl |
| 140 | OH | -O-CH(C₆H₅)-O- | | OH | 3-pyridyl |

[Table 8]

**Table 8**

| Reference Compound No. | R₁ | R₂ | R₃ | R₄ | Het₁ |
|---|---|---|---|---|---|
| 141 | OH | -O-CH(C₆H₅)-O- | | OCOCH₃ | 3-pyridyl |
| 142 | OH | -O-CH(OCH₃)-O- | | OCO(CH₂)₃CH₃ | 3-pyridyl |
| 143 | OH | -O-CH(C₆H₅)-O- | | OCO(CH₂)₃CH₃ | 3-pyridyl |
| 144 | OH | -O-CH(3-CH₃-C₆H₄)-O- | | OH | 3-pyridyl |
| 145 | OH | -O-CH(3-CH₃-C₆H₄)-O- | | OCO(CH₂)₃CH₃ | 3-pyridyl |
| 146 | OH | -O-CH(2-CH₃-C₆H₄)-O- | | OH | 3-pyridyl |
| 147 | OH | -O-CH(4-CH₃-C₆H₄)-O- | | OCO(CH₂)₃CH₃ | 3-pyridyl |
| 148 | OH | -O-CH(3-F-C₆H₄)-O- | | OH | 3-pyridyl |
| 149 | OH | -O-CH(2-F-C₆H₄)-O- | | OCO(CH₂)₃CH₃ | 3-pyridyl |
| 150 | OH | -O-CH(4-F-C₆H₄)-O- | | OCO(CH₂)₃CH₃ | 3-pyridyl |
| 151 | OH | -O-CH(4-NO₂-C₆H₄)-O- | | OH | 3-pyridyl |
| 152 | OH | -O-CH(4-NO₂-C₆H₄)-O- | | OCO(CH₂)₃CH₃ | 3-pyridyl |
| 153 | OH | -O-CH(4-OCH₃-C₆H₄)-O- | | OH | 3-pyridyl |
| 154 | OH | -O-CH(4-OCH₃-C₆H₄)-O- | | OCO(CH₂)₃CH₃ | 3-pyridyl |
| 155 | OH | -O-C(spiro-c-C₅H₈)-O- | | OH | 3-pyridyl |
| 156 | OH | -O-C(spiro-c-C₅H₈)-O- | | OCO(CH₂)₃CH₃ | 3-pyridyl |
| 157 | OH | -O-C(spiro-c-C₆H₁₀)-O- | | OH | 3-pyridyl |
| 158 | OH | -O-C(spiro-c-C₆H₁₀)-O- | | OCO(CH₂)₃CH₃ | 3-pyridyl |
| 159 | OH | -O-CO-O- | | OH | 3-pyridyl |
| 160 | OH | -O-CO-O- | | OCO-1-imidazolyl | 3-pyridyl |

[Table 9]

**Table 9**

| Reference Compound No. | R₁ | R₂ | | R₃ | R₄ | Het₁ |
|---|---|---|---|---|---|---|
| 161 | OH | -O-CO-O- | | | OCO(CH₂)₃CH₃ | 3-pyridyl |
| 162 | OCOCH₃ | OCOCH₃ | OCOCH₃ | | OCOCH₃ | 3-pyridyl |
| 163 | OCOCH₃ | OCOCH₃ | OCOCH₃ | | OH | 3-pyridyl |
| 164 | OCOCH₃ | OCOCH₃ | OCO(CH₂)₂CH₃ | | OCOCH₃ | 3-pyridyl |
| 165 | OCOCH₃ | OH | OH | | OCOCH₃ | 3-pyridyl |
| 166 | OCOCH₃ | OCOCH₂CH₃ | OCOCH₂CH₃ | | OCOCH₂CH₃ | 3-pyridyl |
| 167 | OCOCH₂CH₃ | OCOCH₂CH₃ | OCOCH₂CH₃ | | OCOCH₂CH₃ | 3-pyridyl |
| 168 | OCOCH₂CH₃ | OCOCH₃ | OCOCH₃ | | OCOCH₃ | 3-pyridyl |
| 169 | OCO(CH₂)₃CH₃ | OCOCH₃ | OCOCH₃ | | OCOCH₃ | 3-pyridyl |
| 170 | OCO(CH₂)₃CH₃ | OCOCH₃ | OCOCH₃ | | OCO(CH₂)₃CH₃ | 3-pyridyl |
| 171 | OCO(CH₂)₂CH₃ | OCOCH₃ | OCOCH₃ | | OCOCH₃ | 3-pyridyl |
| 172 | OCH₃ | OCOCH₃ | OCOCH₃ | | OCOCH₃ | 3-pyridyl |
| 173 | H(=) | OSO₂CH₃ | OSO₂CH₃ | | OH | 3-pyridyl |
| 174 | H(=) | OCOC₆H₅ | OSO₂CH₃ | | OCOCH₃ | 3-pyridyl |
| 175 | H(=) | OH | OH | | OCOCH₃ | 3-pyridyl |
| 176 | H(=) | OCOCH₃ | OCOCH₃ | | =O | 3-pyridyl |
| 177 | H(=) | -O-CH(C₆H₅)-O- | | | OCOCH₃ | 3-pyridyl |
| 178 | H(=) | -O-CH(CH(CH₃)₂)-O- | | | OH | 3-pyridyl |
| 179 | H(=) | -O-CH(4-NO₂-C₆H₄)-O- | | | OH | 3-pyridyl |
| 180 | H(=) | OCOCH₃ | | OCOCH₃ | OCOCH₃ | 3-pyridyl |

[Table 10]

**Table 10**

| Reference Compound No. | R₁ | R₂ | R₃ | R₄ | Het₁ |
|---|---|---|---|---|---|
| 181 | H(=) | OH | OH | OH | 3-pyridyl |
| 182 | H(=) | OCOCH₃ | OCOCH₃ | OH | 3-pyridyl |
| 183 | H(=) | OCOCH₃ | OCOCH₃ | OCH₂SCH₃ | 3-pyridyl |
| 184 | H(=) | OCOCH₃ | OCOCH₃ | OCH₂OCH₃ | 3-pyridyl |
| 185 | H(=) | OCOCH₃ | OCOCH₃ | OCO(CH₂)₃CH₃ | 3-pyridyl |
| 186 | H(=) | OCOCH₃ | OCOCH₃ | OCO(CH₂)₂Ph | 3-pyridyl |
| 187 | H(=) | OCOCH₃ | OSO₂CH₃ | OCOCH₃ | 3-pyridyl |
| 188 | H(=) | OCOCH₂CH₃ | OCOCH₂CH₃ | OCOCH₂CH₃ | 3-pyridyl |
| 189 | H(=) | OCOCH₂CH₃ | OCOCH₂CH₃ | OH | 3-pyridyl |
| 190 | H(=) | OH | OSO₂CH₃ | OH | 3-pyridyl |
| 191 | H(=) | OH | OH | OCO(CH₂)₃CH₃ | 3-pyridyl |
| 192 | H(=) | -O-C(CH₃)₂-O- | | OH | 3-pyridyl |
| 193 | H(=) | -O-C(CH₃)₂-O- | | OCO(CH₂)₃CH₃ | 3-pyridyl |
| 194 | H(=) | -O-CH(C₆H₅)-O- | | OH | 3-pyridyl |
| 195 | H(=) | -O-CH(C₆H₅)-O- | | OCO(CH₂)₃CH₃ | 3-pyridyl |
| 196 | H(=) | -O-CH(4-OCH₃-C₆H₄)-O- | | OH | 3-pyridyl |
| 197 | H(=) | -O-CH(C₂H₅)-O- | | OH | 3-pyridyl |
| 198 | H(=) | -O-CH(C(CH₃)₂)-O- | | OH | 3-pyridyl |
| 199 | H(=) | -O-CH(CH₂C₆H₅)-O- | | OH | 3-pyridyl |
| 200 | =O | OH | OH | OH | 3-pyridyl |

[Table 11]

**Table 11**

| Compound No. | R₁ | R₂ | R₃ | R₄ | Het₁ |
|---|---|---|---|---|---|
| Reference compound 201 | =O | OCOCH₃ | OCOCH₃ | =O | 3-pyridyl |
| Reference compound 202 | =O | OCOCH₃ | OCOCH₃ | OH | 3-pyridyl |
| Reference compound 203 | =O | OCOCH₃ | OCOCH₃ | OCOCH₃ | 3-pyridyl |
| Reference compound 204 | =O | OCOCH₂CH₃ | OCOCH₂CH₃ | OCOCH₂CH₃ | 3-pyridyl |
| 205 | OH | OCOCH₂CH₃ | OCOCH₂CH₃ | OCO-(3-Pyridyl) | 3-pyridyl |
| Reference compound 206 | OH | OCOCH₂CH₃ | OCOCH₂CH₃ | OCOCH(CH₃)₂ | 3-pyridyl |
| Reference compound 207 | OH | OCOCH₂CH₃ | OCOCH₂CH₃ | OCOC(CH₃)₃ | 3-pyridyl |
| 208 | OH | OCOCH₂CH₃ | OCOCH₂CH₃ | OCO-(4-CF₃-C₆H₄) | 3-pyridyl |
| 209 | OH | OCOCH₂CH₃ | OCOCH₂CH₃ | OCO-(1-imidazolyl) | 3-pyridyl |
| Reference compound 210 | OH | OCOCH₂CH₃ | OCOCH₂CH₃ | OCONH(CH₂)₂CH₃ | 3-pyridyl |
| 211 | OH | OCOCH₂CH₃ | OCOCH₂CH₃ | O-(2-tetrahydropyranyl) | 3-pyridyl |
| 212 | OH | OCOCH₂CH₃ | OCOCH₂CH₃ | OCO-(6-Cl-3-pyridyl) | 3-pyridyl |
| 213 | OH | OCOCH₂CH₃ | OCOCH₂CH₃ | OCO-c-C₃H₅ | 3-pyridyl |
| 214 | OH | OCOCH₂CH₃ | OCOCH₂CH₃ | OCO-c-C₄H₇ | 3-pyridyl |
| Reference compound 215 | OH | OCOCH₂CH₃ | OCOCH₂CH₃ | OCOCH=CH | 3-pyridyl |
| 216 | OH | OCOCH₂CH₃ | OCOCH₂CH₃ | OCO-(4-pyridyl) | 3-pyridyl |
| 217 | OH | OCOCH₂CH₃ | OCOCH₂CH₃ | OCO-(2-pyridyl) | 3-pyridyl |
| 218 | OH | OCO-c-C₃H₅ | OCO-c-C₃H₅ | OCO-c-C₃H₅ | 3-pyridyl |
| 219 | OH | OCO-c-C₄H₇ | OCO-c-C₄H₇ | OCO-c-C₄H₇ | 3-pyridyl |
| Reference compound 220 | OH | OCOC₆H₅ | OCOC₆H₅ | OCOC₆H₅ | 3-pyridyl |

[Table 12]

**Table 12**

| Compound No. | R₁ | R₂ | R₃ | R₄ | Het₁ |
|---|---|---|---|---|---|
| 221 | OH | OCOCH₂CH₃ | OCOCH₂CH₃ | OCO-(6-CF₃-3-pyridyl) | 3-pyridyl |
| 222 | OH | OCOCH₂CH₃ | OCOCH₂CH₃ | OCO-(4-CF₃-3-pyridyl) | 3-pyridyl |
| Reference compound 223 | OH | OCOCH₂CF₃ | OCOCH₂CF₃ | OCOCH₂CF₃ | 3-pyridyl |
| Reference compound 224 | OH | OCOCH₂CH₃ | OCOCH₂CH₃ | OCOCH₂CF₃ | 3-pyridyl |
| Reference compound 225 | =O | OCOCH₂CH₃ | OCOCH₂CH₃ | OCOCH₂CH₃ | 6-Cl-3-pyridyl |
| 226 | OH | OCOCH₂CH₃ | OCOCH₂CH₃ | OCOCH₂CH₃ | 6-Cl-3-pyridyl |
| 227 | OH | OCOCH₂CH₃ | OCOCH₂CH₃ | OCO-(3-F-4-pyridyl) | 3-pyridyl |
| 228 | OH | OCOCH₂CH₃ | OCOCH₂CH₃ | OCO-(3-Cl-4-pyridyl) | 3-pyridyl |
| 229 | OH | OCOCH₂CH₃ | OCOCH₂CH₃ | OCO-(3-CH₃-2-pyridyl) | 3-pyridyl |
| Reference compound 230 | OH | OCOCH₂CH₃ | OCOCH₂CH₃ | OCO-(3-COC₆H₅-2-pyridyl) | 3-pyridyl |
| Reference compound 231 | OH | OCOCH₂CH₃ | OCOCH₂CH₃ | OCO-(3-OCH₂CH₂CH₃ -2-pyridyl) | 3-pyridyl |
| 232 | OH | OCOCH₂CH₃ | OCOCH₂CH₃ | OCO-(6-F-3-pyridyl) | 3-pyridyl |
| 233 | OH | OCO-C-C₅H₉ | OCO-c-C₅H₉ | OCO-c-C₅H₉ | 3-pyridyl |
| 234 | OH | OCO-c-C₆H₁₁ | OCO-c-C₆H₁₁ | OCO-c-C₆H₁₁ | 3-pyridyl |
| Reference compound 235 | OH | OCOCH₂CN | OCOCH₂CN | OCOCH₂CN | 3-pyridyl |
| Reference compound 236 | OCOCH₂-c-C₃H₅ | OCOCH₂-c-C₃H₅ | OCOCH₂-c-C₃H₅ | OCOCH₂-c-C₃H₅ | 3-pyridyl |
| 237 | OH | OCOCH₂-c-C₃H₅ | OCOCH₂-c-C₃H₅ | OCOCH₂-c-C₃H₅ | 3-pyridyl |
| Reference compound 238 | OH | OCO-(1-CH₃-2,2-di F-c-C₃H₂) | OCO-(1-CH₃-2,2-di F-c-C₃H₂) | OCO-(1-CH₃-2,2-diF-c-C₃H₂) | 3-pyridyl |
| 239 | OH | OCOCH₂CH₃ | OCOCH₂CH₃ | OCO-(4-CH₃-3-pyridyl) | 3-pyridyl |
| 240 | OH | OCOCH₂CH₃ | OCOCH₂CH₃ | OCO-(4-Cl-3-pyridyl) | 3-pyridyl |

[Table 13]

**Table 13**

| Compound No. | R₁ | R₂ | R₃ | R₄ | Het₁ |
|---|---|---|---|---|---|
| Reference compound 241 | OH | OCOCH₂CH₃ | OCOCH₂CH₃ | OCO-(4-COOCH₃-3-pyridyl) | 3-pyridyl |
| Reference compound 242 | OH | OCOCH₂CH₃ | OCOCH₂CH₃ | OCO-[5-(CF₃)-thieno[3,2-b]py ridin-6-yl] | 3-pyridyl |
| 243 | OH | OCOCH₂CH₃ | OCOCH₂CH₃ | OCO-(2-CN-C₆H₄) | 3-pyridyl |
| Reference compound 244 | OH | OCOCH₂CH₃ | OCOCH₂CH₃ | OCO-(2-CF₃-C₆H₄) | 3-pyridyl |
| 245 | OH | OCOCH₂CH₃ | OCOCH₂CH₃ | OCO-(2-F-C₆H₄) | 3-pyridyl |
| 246 | OH | OCOCH₂CH₃ | OCOCH₂CH₃ | OCO-(2-NO₂-C₆H₄) | 3-pyridyl |
| 247 | OH | OCOCH₂CH₃ | OCOCH₂CH₃ | OCO-(2-Cl-3-pyridyl) | 3-pyridyl |
| 248 | OH | OCOCH₂CH₃ | OCOCH₂CH₃ | OCO(2-Cl-6-CH₃-3-pyridyl) | 3-pyridyl |
| Reference compound 249 | OH | OCOCH₂CH₃ | OCOCH₂CH₃ | OCH₂OCH₃ | 3-pyridyl |
| 250 | OH | OCO-(2,2-diF-c-C₃H₃) | OCO-(2,2-diF-c-C₃H₃) | OCO-(2,2-diF-c-C₃H₃) | 3-pyridyl |
| Reference compound 251 | OH | OCOCH₂CH₃ | OCOCH₂CH₃ | OCO-(3-SC(CH₃)₃-2-pyridyl) | 3-pyridyl |
| 252 | OH | OCOCH₂CH₃ | OCOCH₂CH₃ | OCO-(3,5-diF-2-pyridyl) | 3-pyridyl |
| 253 | OH | OCOCH₂CH₃ | OCOCH₂CH₃ | OCO-2-pyrazinyl | 3-pyridyl |
| 254 | OH | OCOCH₂CH₃ | OCOCH₂CH₃ | OCO-4-thiazolyl | 3-pyridyl |
| 255 | OH | OCOCH₂CH₃ | OCOCH₂CH₃ | OCO-(3-Cl-2-thienyl) | 3-pyridyl |
| 256 | OH | OCOCH₂CH₃ | OCOCH₂CH₃ | OCO-(6-CH₃-3-pyridyl) | 3-pyridyl |
| 257 | OH | OCOCH₂CH₃ | OCOCH₂CH₃ | OCO-(6-Cl-2-pyridyl) | 3-pyridyl |
| 258 | OH | OCOCH₂CH₃ | OCOCH₂CH₃ | OCO-(6-F-2-pyridyl) | 3-pyridyl |
| Reference compound 259 | OH | OCOCH₂CH₃ | OCOCH₂CH₃ | OCO-(1-CH₃-1H-indolyl) | 3-pyridyl |
| 260 | OH | OCOCH₂CH₃ | OCOCH₂CH₃ | OCO-(3-Cl-2-pyridyl) | 3-pyridyl |

[Table 14]

**Table 14**

| Compound No. | R₁ | R₂ | R₃ | R₄ | Het₁ |
|---|---|---|---|---|---|
| 261 | OH | OCO-c-C₃H₅ | OCO-c-C₃H₅ | OH | 3-pyridyl |
| 262 | OH | OCOCH₂CH₃ | OCOCH₂CH₃ | OCO-(2-F-3-pyridyl) | 3-pyridyl |
| 263 | OH | OCOCH₂CH₃ | OCOCH₂CH₃ | OCO-(4-CN-C₆H₄) | 3-pyridyl |
| 264 | OH | OCOCH2CH3 | OCOCH₂CH₃ | OCO-(3-CN-C₆H₄) | 3-pyridyl |
| 265 | OH | OCOCH₂CH₃ | OCOCH₂CH₃ | OCO-(3-CF₃-C₆H₄) | 3-pyridyl |
| Reference compound 266 | OH | OCOCH₂CH₃ | OCOCH₂CH₃ | OCOCH₂(2-pyridyl) | 3-pyridyl |
| Reference compound 267 | OH | OCOCH₂CH₃ | OCOCH₂CH₃ | OCOCH₂(3-pyridyl) | 3-pyridyl |
| Reference compound 268 | OH | OCOCH₂CH₃ | OCOCH₂CH₃ | OCOCH₂S(4-pyridyl) | 3-pyridyl |
| 269 | OH | OCO-c-C₃H₅ | OCO-c-C₃H₅ | OCO-(2-CN-C₆H₄) | 3-pyridyl |
| 270 | OH | OCO-c-C₃H₅ | OCO-c-C₃H₅ | OCO(4-CF₃-3-pyrid yl) | 3-pyridyl |
| 271 | OH | OCO-c-C₃H₅ | OCO-c-C₃H₅ | OCO(3-Cl-2-pyridyl) | 3-pyridyl |
| Reference compound 272 | OH | -O-CH(C₆H₅)-O- | | =O | 3-pyridyl |
| Reference compound 273 | OH | -O-CH(4-OCH₃-C₆H₄)-O- | | =O | 3-pyridyl |
| Reference compound 274 | OCO(CH₂)₃C H₃ | -O-CO-O- | | OCO(CH₂)₃CH3 | 3-pyridyl |
| Reference compound 275 | OCOCH₃ | -O-CH(C₆H₅)-O- | | OCOCH₃ | 3-pyridyl |
| Reference compound 276 | =O | -O-CH(4-OCH₃-C₆H₄)-O- | | OH | 3-pyridyl |

### Production process

The compositon according to the present invention can be prepared by mixing the compound represented by formula (Ia), or (Ib) as active ingredient with an agriculturally and horticulturally acceptable carrier. The compound represented by formula (Ia), or (Ib) according to the present invention can be produced according to the following procedure.

Among the compounds according to the present invention, the compounds represented by formula (II) can be synthesized by the method described in Japanese Patent Laid-Open Publication No. 259569/1996, Japanese Patent Laid-Open Publication No. 269062/1996, Japanese Patent Laid-Open Publication No. 269065/1996, or Journal of Antibiotics (1997), 50(3), pp. 229-36. When pyripyropene A is used as a starting material, pyripyropene A, produced by the method described in Journal of Society of Synthetic Organic Chemistry, Japan (1998), Vol. 56, No. 6, pp. 478-488 or WO 94/09417, may be used as the starting material. 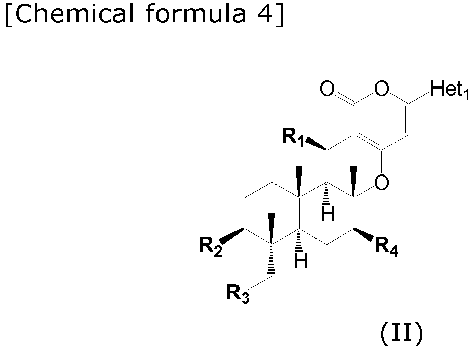 wherein
R₁ represents hydroxyl, and
R₂, R₃ and R₄ are as defined in formula (Ia) or (Ib).

Further, among the compounds according to the present invention, the compounds represented by formula (III) can be synthesized by the method described in Japanese Patent Laid-Open Publication No. 269063/1996, or Japanese Patent Laid-Open Publication No. 269066/1996. 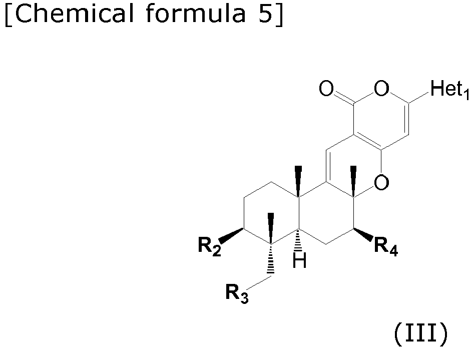 wherein R₂, R₃ and R₄ are as defined in formula (Ia) or (Ib).

### Use

Insect species against which pyripyropene derivatives of formula (I) or (Ib) according to the present invention have control effect include: lepidopteran pests, for example, Spodoptera litura, Mamestra brassicae, Pseudaletia separata, green caterpillar, Plutella xylostella, Spodoptera exigua, Chilo suppressalis, Cnaphalocrocis medinalis, Tortricidae, Carposinidae, Lyonetiidae, Lymantriidae, pests belonging to the genus Agrotis spp., pests belonging to the genus Helicoverpa spp., and pests belonging to the genus Heliothis spp.; hemipteran pests, for example, Aphidoidea including Aphididae, Adelgidae and Phylloxeridae such as Myzus persicae, Aphis gossypii, Aphis fabae, Aphis maidis (corn-leaf aphid), Acyrthosiphon pisum, Aulacorthum solani, Aphis craccivora, Macrosiphum euphorbiae, Macrosiphum avenae, Metopolophium dirhodum, Rhopalosiphum padi, Schizaphis graminum, Brevicoryne brassicae, Lipaphis erysimi, Aphis citricola, Rosy apple aphid, Eriosoma lanigerum, Toxoptera aurantii, and Toxoptera citricidus; Deltocephalidae such as Nephotettix cincticeps, Delphacidae such as Laodelphax striatellus, Nilaparvata lugens, and Sogatella furcifera; Pentatomidae such as Eysarcoris ventralis, Nezara viridula, and Trigonotylus coelestialium; Aleyrodidae such as Bemisia argentifolii, Bemisia tabaci, and Trialeurodes vaporariorum; Diaspididae, Margarodidae, Ortheziidae, Aclerdiae, Dactylopiidae, Kerridae, Pseudococcidae, Coccidae, Eriococcidae, Asterolecaniidae, Beesonidae, Lecanodiaspididae, or Cerococcidae, such as Pseudococcus comstocki and Planococcus citri Risso; Coleoptera pests, for example, Lissorhoptrus oryzophilus, Callosobruchuys chienensis, Tenebrio molitor, Diabrotica virgifera virgifera, Diabrotica undecimpunctata howardi, Anomala cuprea, Anomala rufocuprea, Phyllotreta striolata, Aulacophora femoralis, Leptinotarsa decemlineata, Oulema oryzae, Carposinidae, and Cerambycidae; Acari, for example, Tetranychus urticae, Tetranychus kanzawai, and Panonychus citri; Hymenopteran pests, for example, Tenthredinidae; Orthopteran pests, for example, Acrididae; Dipteran pests, for example, Muscidae and Agromyzidae; Thysanopteran pests, for example, Thrips palmi and Frankliniella occidentalis; Plant Parasitic Nematodes, for example, Meloidogyne hapla, Pratylenchus spp., Aphelenchoides besseyi and Bursaphelenchus xylophilus; and parasites of animals, for example, Siphonaptera, Anoplura, mites such as Boophilus microplus, Haemaphysalis longicornis, Rhipicephalus sanguineus, and Scarcoptes scabiei. Preferred are hemipteran pests.

The compound represented by formula (Ia) accordingly to the present invention has significant control effect against hemipteran pests. Preferred hemipteran pests are selected from Aphidoidea such as Aphididae, Adelgidae, and Phylloxeridae, particularly preferably Aphididae; Coccoidea such as Diaspididae, Margarodidae, Ortheziidae, Aclerdiae, Dactylopiidae, Kerridae, Pseudococcidae, Coccidae, Eriococcidae, Asterolecaniidae, Beesonidae, Lecanodiaspididae, and Cerococcidae; and Aleyrodidae. More preferred are Myzus persicae, Aphis gossypii, Aphis fabae, Aphis maidis (corn-leaf aphid), Acyrthosiphon pisum, Aulacorthum solani, Aphis craccivora, Macrosiphum euphorbiae, Macrosiphum avenae, Metopolophium dirhodum, Rhopalosiphum padi, Schizaphis graminum, Brevicoryne brassicae, Lipaphis erysimi, Aphis citricola, Rosy apple aphid, Eriosoma lanigerum, Toxoptera aurantii, Toxoptera citricidus, and Pseudococcus comstocki.

The composition according to the present invention can be prescribed in any suitable formulation, such as emulsifiable concentrates, liquid formulations, suspension, wettable powder, flowables, dust, granules, tablets, oil solutions, aerosols, or smoking agents by using suitable agriculturally and horticulturally acceptable carriers. Accordingly, the carrier include solid carriers, liquid carriers, gaseous carriers, surfactants, dispersants and/or other adjuvants for formulations, and the like.

Solid carriers usable herein include, for example, talc, bentonite, clay, kaolin, diatomaceous earth, vermiculite, white carbon, and calcium carbonate.

Examples of liquid carriers include: alcohols, such as methanol, n-hexanol, and ethylene glycol; ketones, such as acetone, methyl ethyl ketone, and cyclohexanone; aliphatic hydrocarbons, such as n-hexane, kerosine, and kerosene; aromatic hydrocarbons, such as toluene, xylene, and methylnaphthalene; ethers, such as diethyl ether, dioxane, and tetrahydrofuran; esters, such as ethyl acetate; nitriles, such as acetonitrile and isobutyronitrile; acid amides, such as dimethylformamide and dimethylacetamide; vegetable oils, such as soy bean oil and cotton seed oil; dimethylsulfoxide; and water.

Gaseous carriers include, for example, LPG, air, nitrogen, carbon dioxide, and dimethyl ether.

Surfactants or dispersants usable, for example, for emulsifying, dispersing, or spreading include, for example, alkylsulfonic esters, alkyl(aryl)sulfonic acid salts, polyoxyalkylene alkyl(aryl) ethers, polyhydric alcohol esters, and lignin sulfonic acid salts.

Adjuvants usable for improving the properties of formulations include, for example, carboxymethylcellulose, gum arabic, polyethylene glycol, and calcium stearate.

The above carriers, surfactants, dispersants, and adjuvant may be used either solely or in combination according to need.

The content of the active ingredient in the formulation is not particularly limited. In general, however, the content of the active ingredient is 1 to 75% by weight for emulsifiable concentrates, 0.3 to 25% by weight for dust, 1 to 90% by weight for wettable powder, and 0.5 to 10% by weight for granules.

The compound represented by formula (Ia), (Ib), or an agriculturally and horticulturally acceptable salt thereof and the above formualtions comprising the same may be applied as such or after dilution to plants or soil. Therefore, according to another aspect of the present invention, there is provided a method for controlling a pest, comprising applying an effective amount of a compound represented by formula (Ia) or (Ib) or an agriculturally and horticulturally acceptable salt thereof to a plant or soil. According to still another aspect of the present invention, there is provided a method for controlling a hemipteran pest, comprising applying an effective amount of a compound represented by formula (Ia) or an agriculturally and horticulturally acceptable salt thereof to a plant or soil. According to a further aspect of the present invention, there is provided a method for controlling a pest, comprising applying an effective amount of a compound represented by formula (Ib) or an agriculturally and horticulturally acceptable salt thereof to a plant or soil. Preferred methods usable for applying the compound or formulation to plants or soil include spreading treatment, soil treatment, surface treatment, and fumigation treatment.

Spreading treatments include, for example, spreading, spraying, misting, atomizing, granule application, and submerged application. Soil treatments include, for example, soil affusion and soil mixing. Examples of surface treatments include, for example, coating, dust coating, and covering. Fumigation treatments include, for example, covering of soil with a polyethylene film after soil injection. Accordingly, the control method according to the present invention comprises a method in which the compound represented by formula (Ia), or (Ib) or a formulation comprising the same is applied by fumigation in a sealed space.

The composition according to the present invention may be used as a mixture or in a combination with, for example, other insecticides, fungicides, miticides, herbicides, plant growth-regulating agents, or fertilizers. Agents which may be mixed or used in combination include those described, for example, in The Pesticide Manual, 13th edition, published by The British Crop Protection Council; and SHIBUYA INDEX, the 10th edition, 2005, published by SHIBUYA INDEX RESEARCH GROUP. More specifically, insecticides usable herein include, for example, organophosphate ester compounds such as acephate, dichlorvos, EPN, fenitrothion, fenamifos, prothiofos, profenofos, pyraclofos, chlorpyrifos-methyl, and diazinon; carbamate compounds such as methomyl, thiodicarb, aldicarb, oxamyl, propoxur, carbaryl, fenobucarb, ethiofencarb, fenothiocarb, pirimicarb, carbofuran, and benfuracarb; nereistoxin derivatives such as cartap and thiocyclam; organochlorine compounds such as dicofol and tetradifon; pyrethroid compounds such as permethrin, tefluthrin, cypermethrin, deltamethrin, cyhalothrin, fenvalerate, fluvalinate, ethofenprox, and silafluofen; benzoylurea compounds such as diflubenzuron, teflubenzuron, flufenoxuron, and chlorfluazuron; juvenile hormone-like compounds such as methoprene; and molting hormone-like compounds such as chromafenozide. Other compounds usable herein include buprofezin, hexythiazox, amitraz, chlordimeform, pyridaben, fenpyroxymate, pyrimidifen, tebufenpyrad, fluacrypyrim, acequinocyl, cyflumetofen, flubendiamide, ethiprole, fipronil, ethoxazole, imidacloprid, chlothianidin, pymetrozine, bifenazate, spirodiclofen, spiromesifen, flonicamid, chlorfenapyr, pyriproxyfene, indoxacarb, pyridalyl, or spinosad, avermectin, milbemycin, organometallic compounds, dinitro compounds, organosulfur compounds, urea compounds, triazine compounds, hydrazine compounds.

The composition according to the present invention may also be used as a mixture or in a combination with microbial pesticides such as BT formulations and entomopathogenic viral agents.

Fungicides usable herein include, for example, strobilurin compounds such as azoxystrobin, kresoxym-methyl, and trifloxystrobin; anilinopyrimidine compounds such as mepanipyrim, pyrimethanil, and cyprodinil; azole compounds such as triadimefon, bitertanol, triflumizole, etaconazole, propiconazole, penconazole, flusilazole, myclobutanil, cyproconazole, tebuconazole, hexaconazole, prochloraz, and simeconazole; quinoxaline compounds such as quinomethionate; dithiocarbamate compounds such as maneb, zineb, mancozeb, polycarbamate, and propineb; phenylcarbamate compounds such as diethofencarb; organochlorine compounds such as chlorothalonil and quintozene; benzimidazole compounds such as benomyl, thiophanate-methyl, and carbendazole; phenylamide compounds such as metalaxyl, oxadixyl, ofurace, benalaxyl, furalaxyl, and cyprofuram; sulfenic acid compounds such as dichlofluanid; copper compounds such as copper hydroxide and oxine-copper; isoxazole compounds such as hydroxyisoxazole; organophosphorus compounds such as fosetyl-aluminium and tolclofos-methyl; N-halogenothioalkyl compounds such as captan, captafol, and folpet; dicarboxyimide compounds such as procymidone, iprodione, and vinchlozolin; benzanilide compounds such as flutolanil and mepronil; morpholine comopounds such as fenpropimorph and dimethomorph; organotin compounds such as fenthin hydroxide, and fenthin acetate; and cyanopyrrole compounds such as fludioxonil and fenpiclonil. Other compounds usable herein include fthalide, fluazinam, cymoxanil, triforine, pyrifenox, fenarimol, fenpropidin, pencycuron, cyazofamid, iprovalicarb, and benthiavalicarb-isopropyl and the like.

According to another aspect of the present invention, there is provided use of a compound represented by formula (Ia) or (Ib) or an agriculturally and horticulturally acceptable salt thereof as a pest control agent. According to still another aspect of the present invention, there is provided use of a compound represented by formula (Ia) or an agriculturally and horticulturally acceptable salt thereof as a hemipteran pest control agent. According to still another aspect of the present invention, there is provided use of a compound represented by formula (Ib) or an agriculturally and horticulturally acceptable salt thereof as a pest control agent.

### [EXAMPLES]

The present invention is further illustrated by the following Examples. The compound Nos. correspond to the compound Nos. in Tables 1 to 14.

### Example 1: Synthesis of compound 73

Compound 76 (890 mg) synthesized by the method described in Japanese Patent Laid-Open Publication No. 259569/1996 was dissolved in an 80% aqueous methanol solution. Next, 1,8-diazabicyclo[5.4.0]-undeca-7-ene (216 mg) was added to the solution, and the mixture was stirred at room temperature for 1.5 hr. The reaction mixture was added with acetic acid to quench the reaction, and the solvent was removed by evaporation under the reduced pressure. Water was added to the precipitated crystal, followed by extraction with chloroform. The chloroform layer was washed with saturated brine, was dried over anhydrous magnesium sulfate, and the solvent was removed by evaporation under the reduced pressure to give a crude product of compound 73. The crude product was purified by chromatography on silica gel (Mega Bond Elut (Varian), acetone : hexane = 1 : 1) to give compound 73 (451 mg).
Mass spectrometric data (FAB⁺): 570(M+H)⁺

### Example 2: Synthesis of compound 218

Compound 102 (30 mg) synthesized by the method described in Japanese Patent Laid-Open Publication No. 259569/1996 and cyclopropanecarboxylic acid (112 mg) were dissolved in anhydrous N,N-dimethylformamide (2 ml), and 1-ethyl-3-(3-dimethylaminopropyl)-carbodiimide hydrochloride (76 mg) and 4-(dimethylamino)pyridine (32 mg) were added to the solution. The reaction solution was stirred at room temperature for 68 hr and was then poured into water, followed by extraction with ethyl acetate. The ethyl acetate layer was washed with saturated brine and was dried over anhydrous magnesium sulfate, and the solvent was removed by evaporation under the reduced pressure to give a crude product of compound 218. The crude product was purified by preparative thin-layer chromatography (Merck Silica Gel 60 F₂₅₄ 0.5mm, acetone : hexane = 1 : 1) to give compound 218 (33 mg).
Mass spectrometric data (FAB⁺): 662(M+H)⁺

### Example 3: Synthesis of compound 261

Compound 218 (1.07 g) prepared in Example 2 was dissolved in an 80% aqueous methanol solution. 1,8-Diazabicyclo[5.4.0]-undeca-7-ene (271 mg) was added to the solution, and the mixture was stirred at room temperature for 24.5 hr. The reaction mixture was added with acetic acid to quench the reaction, and the solvent was removed by evaporation under the reduced pressure. Water was added to the precipitated crystal, followed by extraction with chloroform. The chloroform layer was washed with saturated brine and was dried over anhydrous magnesium sulfate, and the solvent was removed by evaporation under the reduced pressure to give a crude product of compound 261. The crude product was purified by chromatography on silica gel (Mega Bond Elut (Varian), acetone : hexane = 1 : 1) to give compound 261 (233 mg).
Mass spectrometric data (ESI⁺): 594(M+H)⁺

### Example 4: Synthesis of compound 222

Compound 73 (30 mg) prepared in Example 1 and 4-(trifluoromethyl)nicotinic acid (30 mg) was dissolved in anhydrous N,N-dimethylformamide (3 ml). Next, 1-ethyl-3-(3-dimethylaminopropyl)-carbodiimide hydrochloride (15 mg) and 4-(dimethylamino)pyridine (4 mg) were added to the solution, and the reaction solution was stirred at room temperature for 15 hr and was then poured into water, followed by extraction with ethyl acetate. The ethyl acetate layer was washed with saturated brine and was dried over anhydrous magnesium sulfate, and the solvent was removed by evaporation under the reduced pressure to give a crude produce of compound 222. The crude product was purified by preparative thin-layer chromatography (Merck Silica Gel 60 F₂₅₄ 0.5mm, acetone : hexane = 1 : 1) to give compound 222 (19 mg).
Mass spectrometric data (FAB⁺): 743(M+H)⁺

### Example 5: Synthesis of compound 269

Compound 261 (20 mg) prepared in Example 3 and 2-cyanobenzoic acid (30 mg) were dissolved in anhydrous N,N-dimethylformamide (1 ml), and 1-ethyl-3-(3-dimethylaminopropyl)-carbodiimide hydrochloride (26 mg) and 4-(dimethylamino)pyridine (4 mg) were added to the solution. The reaction solution was stirred at room temperature for 12 hr, and the reaction solution was added to water, followed by extraction with ethyl acetate. The ethyl acetate layer was washed with saturated brine and was dried over anhydrous magnesium sulfate. The solvent was removed by evaporation under the reduced pressure to give a crude product of compound 269. The crude product was purified by preparative thin-layer chromatography (Merck Silica Gel 60 F₂₅₄ 0.5mm, acetone : hexane = 1 : 1) to give compound 269 (18 mg).
Mass spectrometric data (ESI⁺): 723 (M+H)⁺

### Example 6: Synthesis of reference compound 225

1,7,11-Trideacetyl-13-oxo-6"-chloropyripyropene A (10 mg) described in Journal of Antibiotics (1997), 50 (3), 229-36 was dissolved in anhydrous N,N-dimethylformamide (1 ml). Triethylamine (24 mg) and 4-(dimethylamino)pyridine (0.5 mg) were added to the solution, and the mixture was stirred at room temperature for 30 min. Thereafter, propionic acid anhydride (8 mg) was added. The reaction solution was stirred at the same temperature for 4 hr. The reaction solution was added to water, and the mixture was extracted with ethyl acetate. The ethyl acetate layer was washed with saturated brine and was dried over anhydrous magnesium sulfate, and the solvent was then removed by evaporation under the reduced pressure to give a crude product of compound 225. The crude product was purified by preparative thin-layer chromatography (Merck Silica Gel 60 F₂₅₄ 0.5mm, acetone : hexane = 1 : 1) to give compound 225 (5.6 mg).
Mass spectrometric data (ESI⁺): 658 (M+H)⁺

### Example 7: Synthesis of compound 226

Compound 225 (10 mg) prepared in Example 6 was dissolved in methanol (1 ml). Cerium(III) chloride heptahydrate (57 mg) and sodium borohydride (6 mg) were added to the solution. The mixture was stirred at 0°C for 7 hr, and water was added to the reaction solution, followed by extraction with ethyl acetate. The ethyl acetate layer was washed with saturated brine and was dried over anhydrous magnesium sulfate, and the solvent was removed by evaporation under the reduced pressure to give a crude product of compound 226. The crude product was purified by preparative thin-layer chromatography (Merck Silica Gel 60 F₂₅₄ 0.5mm, acetone : hexane = 1 : 1) to give compound 226 (8.5 mg).
Mass spectrometric data (ESI⁺): 660 (M+H)⁺

### Example 8: Synthesis of reference compound 273

1,7,11-Trideacetyl-1,11-o-p-methoxybenzylidene pyripyro-pene A (10 mg) described in Japanese Patent Laid-Open Publication No. 269065/1996 was dissolved in anhydrous dichloromethane (0.5 ml), and pyridinium dichromate (PDC) (39 mg) was added to the solution. The reaction solution was stirred at room temperature for 4 hr, and the reaction solution was added to water. The dichloromethane layer was washed with saturated brine, and was dried over anhydrous sodium sulfate, and the solvent was then removed by evaporation under the reduced pressure to give a crude product of compound 273. The crude product was purified by preparative thin-layer chromatography (Merck Silica Gel 60 F₂₅₄ 0.5mm, chloroform : methanol = 12.5 : 1) to give compound 273 (4.4 mg).
Mass spectrometric data (ESI⁺): 574 (M+H)⁺

### Example 9: Synthesis of reference compound 274 1,11-o-Cyclic

carbonate1,7,11-trideacetyl-pyripyropene A (4 mg) described in Japanese Patent Laid-Open Publication No. 269065/1996 was dissolved in anhydrous dichloromethane (1 ml). Triethylamine (5 µl) and 4-(dimethylamino)pyridine (1 mg) were added to the solution. The reaction solution was stirred at room temperature for 30 min, and valeric acid anhydride (5 µl) was added thereto. Next, the reaction solution was stirred at room temperature for 3 hr. The reaction solution was added to water, and the dichloromethane layer was washed with saturated brine and was dried over anhydrous sodium sulfate. The solvent was then removed by evaporation under the reduced pressure to give a crude product of compound 274. The crude product was purified by preparative thin-layer chromatography (Merck Silica Gel 60 F₂₅₄ 0.5mm, chloroform : methanol = 25 : 1) to give compound 274 (0.1 mg).
Mass spectrometric data (ESI⁺): 652 (M+H)⁺

### Example 10

Compounds shown in Tables 15 to 17 were synthesized using starting materials, reaction reagents 1 and 2 and solvents described in these tables. Further, the ¹H-NMR data about some of the compounds in Tables 15 to 17 was described in Tables 18 to 29. In addition, CDCl₃ was used as the solvent for the ¹H-NMR measurement. Tetramethylsilane was used as a standard substance for the ¹H-NMR measurement.
Of note, compounds 206, 207, 210, 215, 220, 223, 224, 230, 231, 235, 236 and 238 in tables 15 and 16 are reference compounds.
[Table 15]

**Table 15**

| Compound No. | Starting material (Compound No.) | Amount | Reaction reagent 1 | Amount | Reaction reagent 2 | Solvent | Yield | Mass spectrometric data | |
|---|---|---|---|---|---|---|---|---|---|
| | | | | | | | | Measuring Method | Data |
| 74 | 73 | 30 mg | acetic anhydride | 32.7 mg | Et₃N 64.0 mg, DMAP 12.8 mg | DMF | 13.6 mg | FAB | 612 (M+H)⁺ |
| 77 | 73 | 30 mg | benzoic acid | 84.8 mg | EDCI 49.2 mg, DMAP 46.4 mg | DMF | 36.4 mg | FAB | 674 (M+H)⁺ |
| 91 | 102 | 30 mg | pivalic anhydride | 220 mg | Et₃N 60.0 mg, DMAP 8.0 mg | DMF | 27.7 mg | FAB | 710 (M+H)⁺ |
| 205 | 73 | 30 mg | nicotinic acid | 12.9 mg | EDCI 15.1 mg, DMAP 6.4 mg | DMF | 27.1 mg | FAB | 675 (M+H)⁺ |
| 206 | 73 | 30 mg | isobutyric anhydride | 50.0 mg | Et₃N 64.0 mg, DMAP 12.8 mg | DMF | 11.4 mg | FAB | 640 (M+H)⁺ |
| 207 | 73 | 30 mg | pivalic anhydride | 58.9 mg | Et₃N 64.0 mg, DMAP 12.8 mg | DMF | 23.4 mg | FAB | 654 (M+H)⁺ |
| 208 | 73 | 30 mg | 4-(trifluoromethyl)benzoic anhydride | 114 mg | Et₃N 64.0 mg, DMAP 12.8 mg | DMF | 32.2 mg | FAB | 742 (M+H)⁺ |
| 209 | 73 | 40 mg | 1,1-carbonyldiimidazole | 34.0 mg | - | toluene | 5.1 mg | FAB | 664 (M+H)⁺ |
| 210 | 73 | 30 mg | propylisocyanate | 26.9 mg | Et₃N 64.0 mg, DMAP 12.8 mg | DMF | 3.2 mg | FAB | 655 (M+H)⁺ |
| 211 | 73 | 30 mg | 3,4-dihydro-2H-pyran | 155 mg | pyridine hydrochloride | CH₂Cl₂ | 22.7 mg | FAB | 654 (M+H)⁺ |
| 212 | 73 | 30 mg | 6-chloronicotinic acid | 16.5 mg | EDCI 15.2 mg, DMAP 6.4 mg | DMF | 39.8 mg | FAB | 709 (M+H)⁺ |
| 213 | 73 | 30 mg | cyclopropane carboxylic acid | 27 mg | EDCI 15.2 mg, DMAP 6.4 mg | DMF | 18.2 mg | FAB | 638 (M+H)⁺ |
| 214 | 73 | 30 mg | cyclobutane carboxylic acid | 31 mg | EDCI 15.2 mg, DMAP 6.4 mg | DMF | 14.9 mg | FAB | 652 (M+H)⁺ |
| 215 | 73 | 30 mg | acrylic acid | 22.5 mg | EDCI 15.2 mg, DMAP 6.4 mg | DMF | 5.6 mg | FAB | 624 (M+H)⁺ |
| 216 | 73 | 30 mg | isonicotinic acid | 12.9 mg | EDCI 15.2 mg, DMAP 6.4 mg | DMF | 8.2 mg | FAB | 675 (M+H)⁺ |
| 217 | 73 | 30 mg | picolinic acid | 12.9 mg | EDCI 15.2 mg, DMAP 6.4 mg | DMF | 40.6 mg | FAB | 675 (M+H)⁺ |
| 219 | 102 | 30 mg | cyclobutane carboxylic acid | 131 mg | EDCI 76 mg, DMAP 32 mg | DMF | 38.9 mg | FAB | 704 (M+H)⁺ |
| 220 | 102 | 30 mg | benzoic acid | 160 mg | EDCI 126 mg, DMAP 80 mg | DMF | 37.9 mg | FAB | 770 (M+H)⁺ |
| 221 | 73 | 30 mg | 6-(trifluoromethyl)nicotinic acid | 30 mg | EDCI 15.2 mg, DMAP 6.4 mg | DMF | 35.4 mg | FAB | 743 (M+H)⁺ |
| 223 | 102 | 30 mg | 3,3,3-trifluoropropionic acid | 168 mg | EDCI 126 mg, DMAP 80 mg | DMF | 10.4 mg | FAB | 788 (M+H)⁺ |
| 224 | 73 | 30 mg | 3,3,3-trifluoropropionic acid | 20 mg | EDCI 15.2 mg, DMAP 6.4 mg | DMF | 8.0 mg | FAB | 680 (M+H)⁺ |

[Table 16]

**Table 16**

| Compound No. | Starting material (Compound No.) | Amount | Reaction reagent 1 | Amount | Reaction reagent 2 | Solvent | Yield | Mass spectrometric data | |
|---|---|---|---|---|---|---|---|---|---|
| | | | | | | | | Measuring Method | Data |
| 227 | 73 | 20 mg | 3-fluoro-isonicotinic acid | 15 mg | EDCI 14 mg, DMAP 4 mg | DMF | 5.4 mg | FAB | 693 (M+H)⁺ |
| 228 | 73 | 20 mg | 3-chloro-isonicotinic acid | 17 mg | EDCI 14 mg, DMAP 4 mg | DMF | 7.8 mg | FAB | 709 (M+H)⁺ |
| 229 | 73 | 20 mg | 3-methylpicolinic acid | 14 mg | EDCI 28 mg, DMAP 8 mg | DMF | 16.7 mg | FAB | 689 (M+H)⁺ |
| 230 | 73 | 20 mg | 3-benzoyl-2-pyridine carboxylic acid | 48 mg | EDCI 28 mg, DMAP 8 mg | DMF | 16.4 mg | FAB | 779 (M+H)⁺ |
| 231 | 73 | 20 mg | 3-n-propoxy picolinic acid | 38 mg g | EDCI 28 mg, DMAP 8 mg | DMF | 17.3 mg | FAB | 733 (M+H)⁺ |
| 232 | 73 | 20 mg | 6-fluoro nicotinic acid | 30 mg g | EDCI 28 mg, DMAP 8 mg | DMF | 5.3 mg | FAB | 693 (M+H)⁺ |
| 233 | 102 | 20 mg | cyclopentane carboxylic acid | 99 mg | EDCI 84 mg, DMAP 5 mg | DMF | 28.3 mg | FAB | 746 (M+H)⁺ |
| 234 | 102 | 20 mg | cyclohexane carboxylic acid | 112 mg | EDCI 84 mg, DMAP 5 mg | DMF | 21.5 mg | FAB | 788 (M+H)⁺ |
| 235 | 102 | 20 mg | cyano acetic acid | 74 mg | EDCI 84 mg, DMAP 5 mg | DMF | 3.3 mg | FAB | 659 (M+H)⁺ |
| 236 | 102 | 20 mg | cyclopropylacetic acid | 87 mg | EDCI 84 mg, DMAP 5 mg | DMF | 16.7 mg | FAB | 786 (M+H)⁺ |
| 237 | 102 | 20 mg | cyclopropylacetic acid | 87 mg | EDCI 84 mg, DMAP 5 mg | DMF | 8.2 mg | FAB | 704 (M+H)⁺ |
| 238 | 102 | 20 mg | 2,2-difluoro-1-methylcyclopropanecarboxylic acid | 118 mg | EDCI 84 mg, DMAP 5 mg | DMF | 6.1 mg | FAB | 812 (M+H)⁺ |
| 239 | 73 | 20 mg | 4-methylnicotinic acid | 36 mg | EDCI 28 mg, DMAP 8 mg | DMF | 16.1 mg | FAB | 689 (M+H)⁺ |
| 240 | 73 | 20 mg | 4-chloronicotinic acid | 33 mg | EDCI 28 mg, DMAP 8 mg | DMF | 13.8 mg | FAB | 709 (M+H)⁺ |
| 241 | 73 | 20 mg | (4-methoxycarbonyl)nicotinic acid | 38 mg | EDCI 28 mg, DMAP 8 mg | DMF | 18.8 mg | FAB | 733 (M+H)⁺ |
| 242 | 73 | 20 mg | 5-(trifluoromethyl)thieno[3,2-b]pyrdine-6-carboxylic acid | 38 mg | EDCI 28 mg, DMAP 8 mg | DMF | 20.3 mg | FAB | 799 (M+H)⁺ |
| 243 | 73 | 20 mg | 2-cyanobenzoic acid | 31 mg | EDCI 28 mg, DMAP 8 mg | DMF | 6.6 m g | FAB | 699 (M+H)⁺ |
| 244 | 73 | 20 mg | 2-(trifluoromethyl)benzoic acid | 40 mg | EDCI 28 mg, DMAP 8 mg | DMF | 10.2 mg | FAB | 742 (M+H)⁺ |
| 245 | 73 | 20 mg | 2-fluorobenzoic acid | 29 mg | EDCI 28 mg, DMAP 8 mg | DMF | 16.1 mg | FAB | 692 (M+H)⁺ |
| 246 | 73 | 20 mg | 2-nitrobenzoic acid | 35 mg | EDCI 28 mg, DMAP 8 mg | DMF | 9.8 mg | FAB | 719 (M+H)⁺ |
| 247 | 73 | 20 mg | 2-chloronicotinic acid | 33 mg | EDCI 28 mg, DMAP 8 mg | DMF | 13.1 mg | FAB | 709 (M+H)⁺ |

[Table 17]

**Table 17**

| Compound No. | Starting material (Compound No.) | Amount | Reaction reagent 1 | Amount | Reaction reagent 2 | Solvent | Yield | Mass spectrometric data | |
|---|---|---|---|---|---|---|---|---|---|
| | | | | | | | | Measuring Method | Data |
| 248 | 73 | 20 mg | 2-chloro-6-methylnicotinic acid | 36 mg | EDCI 28 mg, DMAP 8 mg | DMF | 17.2 mg | FAB | 723 (M+H)⁺ |
| 249 | 73 | 20 mg | methoxymethylbromide | 31 mg | [(CH₃)₂CH]₂NEt 18 mg | DMF | 1.2 mg | ESI | 614 (M+H)⁺ |
| 250 | 102 | 20 mg | 2,2-difluorocyclopropanecarboxylic acid | 106 mg | EDCI 84 mg, DMAP 5 mg | DMF | 23.2 mg | ESI | 770 (M+H)⁺ |
| 251 | 73 | 20 mg | 3-tert-buthylthio-2-carboxypiridine | 44 mg | EDCI 28 mg, DMAP 8 mg | DMF | 7.6 mg | ESI | 763 (M+H)⁺ |
| 252 | 73 | 20 mg | 3,5-difluoropyridine-2-carboxylic acid | 33 mg | EDCI 28 mg, DMAP 8 mg | DMF | 10.9 mg | ESI | 711 (M+H)⁺ |
| 253 | 73 | 20 mg | pyrazinecarboxylic acid | 26 mg | EDCI 28 mg, DMAP 8 mg | DMF | 10.9 mg | ESI | 676 (M+H)⁺ |
| 254 | 73 | 20 mg | 4-thiazolecarboxylic acid | 27 mg | EDCI 28 mg, DMAP 8 mg | DMF | 18.5 mg | ESI | 681 (M+H)⁺ |
| 255 | 73 | 20 mg | 3-chlorothiophene-2-carboxylic acid | 34 mg | EDCI 28 mg, DMAP 8 mg | DMF | 15.8 mg | ESI | 714 (M+H)⁺ |
| 256 | 73 | 20 mg | 6-methylnicotinic acid | 29 mg | EDCI 28 mg, DMAP 8 mg | DMF | 15.1 mg | ESI | 689 (M+H)⁺ |
| 257 | 73 | 20 mg | 6-chloropyridine-2-carboxylic acid | 33 mg | EDCI 28 mg, DMAP 8 mg | DMF | 12.7 mg | ESI | 709 (M+H)⁺ |
| 258 | 73 | 20 mg | 6-fluoropyridine-2-carboxylic acid | 30 mg | EDCI 28 mg, DMAP 8 mg | DMF | 14.4 mg | ESI | 693 (M+H)⁺ |
| 259 | 73 | 20 mg | 1-methylindole-2-carboxylic acid | 37 mg | EDCI 28 mg, DMAP 8 mg | DMF | 18.8 mg | ESI | 727 (M+H)⁺ |
| 260 | 73 | 20 mg | 3-chloropyridine-2-carboxylic acid | 33 mg | EDCI 28 mg, DMAP 8 mg | DMF | 14.6 mg | ESI | 709 (M+H)⁺ |
| 262 | 73 | 20 mg | 2-fluoronicotinic acid | 30 mg | EDCI 28 mg, DMAP 8 mg | DMF | 9.9 mg | ESI | 693 (M+H)⁺ |
| 263 | 73 | 20 mg | 4-cyanobenzoic acid | 31 mg | EDCI 28 mg, DMAP 8 mg | DMF | 14.0 mg | ESI | 699 (M+H)⁺ |
| 264 | 73 | 20 mg | 3-cyanobenzoic acid | 31 mg | EDCI 28 mg, DMAP 8 mg | DMF | 16.9 mg | ESI | 699 (M+H)⁺ |
| 265 | 73 | 20 mg | 3-(trifluoromethyl)benzoic acid | 40 mg | EDCI 28 mg, DMAP 8 mg | DMF | 14.3 mg | ESI | 742 (M+H)⁺ |
| 266 | 73 | 20 mg | 2-pyridylacetic acid | 36 mg | EDCI 28 mg, DMAP 8 mg | DMF | 11.7 mg | ESI | 689 (M+H)⁺ |
| 267 | 73 | 20 mg | 3-pyridylacetic acid | 36 mg | EDCI 28 mg, DMAP 8 mg | DMF | 8.6 mg | ESI | 689 (M+H)⁺ |
| 268 | 73 | 20 mg | (4-pyridylthio)acetic acid | 36 mg | EDCI 28 mg, DMAP 4 mg | DMF | 16.5 mg | ESI | 721 (M+H)⁺ |
| 270 | 261 | 20 mg | 4-(trifluoromethyl)nicotinic acid | 39 mg | EDCI 26 mg, DMAP 4 mg | DMF | 8.3 mg | ESI | 767 (M+H)⁺ |
| 271 | 261 | 20 mg | 3-chloropyridine-2-carboxylic acid | 32 mg | EDCI 26 mg, DMAP 4 mg | DMF | 14.5 mg | ESI | 733 (M+H)⁺ |

[Table 18]

**Table 18**

| Compound No. | ¹H-NMR *δ* (ppm) |
|---|---|
| 73 | 0.91 (3H, s), 1.13 (3H, t, J = 5.1 H z), 1.14 (3H, t, J = 5.1 H z), 1.26 (1H, s), 1.32-1.40 (1H, m), 1.42 (3H, s), 1.45 (1H, d, J = 2.7 Hz), 1.49-1.51 (2H, m), 1.66 (3H, s), 1.81-1.91 (2H, m), 2.13-2.18 (1H, m), 2.24-2.37 (4H, m), 2.90 (1H, m), 3.79 (3H, m), 4.80 (1H, dd, J = 3.5, 7.6 H z), 4.99-5.00 (1H, m), 6.52 (1H, s), 7.42 (1H, dd, J = 3.5, 5.4 Hz), 8.11 (1H, dt, J = 1.4, 5.4 Hz), 8.70 (1H, d, J = 2.4 Hz), 9.00 (1H, s) |
| 77 | 0.92 (3H, s), 1.14 (3H, t, J = 7.6 H z), 1.20 (3H, t, J = 7.6 H z), 1.26 (1H, s), 1.37-1.46 (1H, m), 1.51 (3H, s), 1.62 (1H, d, J = 3.8 Hz), 1.68-1.82 (2H, m), 1.87 (3H, s), 1.91-2.00 (2H, m), 2.18-2.23 (1H, m), 2.33 (2H, q, J = 7.6 H z), 2.43 (2H, dq, J = 1.4, 7.6 Hz), 2.97 (1H, s), 3.70 (1H, d, J = 11.9 Hz), 3.84 (1H, d, J = 11.9 Hz), 4.83 (1H, dd, J = 5.1, 11.1 Hz), 5.05 (1H, d, J = 4.3 H z), 5.27 (1H, dd, J = 4.6, 11.1 H z), 6.45 (1H, s), 7.39-7.66 (4H, m), 8.05-8.13 (3H, m), 8.70 (1H, d, J = 4.6 Hz), 9.00 (1H, s) |
| 74 | 0.90 (3H, s), 1.12 (3H, t J = 7.8 Hz), 1.13 (3H, t J = 7.8 Hz), 1.19 (1H, s), 1.25-1.34 (1H, m), 1.44 (3H, s), 1.53-1.63 (3H, m), 1.69 (3H, s), 1.73-1.90 (2H, m), 2.10 (1H, m), 2.16 (3H, s), 2.33 (2H, dq, J = 2.4, 7.6 H z), 2.36 (2H, dq, J = 3.2, 7.6 H z), 2.87 (1H, m), 3.72 (2H, m), 4.81 (1H, dd, J = 4.6, 11.6 H z), 4.97-5.00 (2H, m), 6.46 (1H, s), 7.40 (1H, dd, J = 4.6, 8.1 H z), 8.10 (1H, m), 8.69 (1H, d, J = 4.9 Hz), 9.00 (1H, s) |
| 205 | 0.92 (3H, s), 1.14 (3H, t, J = 7.6 H z), 1.20 (3H, t, J = 7.6 H z), 1.26 (1H, s), 1.42-1.50 (1H, m), 1.59 (3H, s), 1.61-1.83 (3H, m), 1.85 (3H, s), 1.83-2.00 (2H, m), 2.18-2.23 (1H, m), 2.33 (2H, q, J = 7.6 H z), 2.43 (2H, q, J = 7.6 Hz), 2.94 (1H, m), 3.72 (1H, d, J = 11.9 Hz), 3.82 (1H, d, J = 12.7 H z), 4.83 (1H, dd, J = 4.9, 11.3 H z), 5.03-5.06 (1H, m), 5.27 (1H, dd, J = 4.9, 11.3 H z), 6.42 (1H, s), 7.38 (1H, dd, J = 4.9, 8.1 H z), 7.45 (1H, dd, J = 4.9, 8.1 H z), 8.07 (1H, dt, J = 2.2, 8.1 H z), 8.36 (1H, dt, J = 1.9, 8.1 H z), 8.67 (1H, dd, J = 1.9, 5.1 Hz), 8.83 (1H, dd, J = 1.9, 4.9 Hz), 8.97 (1H, d, J = 1.9 Hz), 9.30 (1H, d, J = 1.9 Hz) |
| 206 | 0.90 (3H, s), 1.13 (6H, t, J = 7.6 H z), 1.19 (1H, s), 1.24 (3H, d, J = 4.6 H z), 1.26 (3H, d, J = 4.6 H z), 1.33-1.38 (1H, m), 1.45 (3H, s), 1.54 (1H, d, J = 3.8 H z), 1.60-1.64 (2H, m), 1.67 (3H, s), 1.75-1.90 (2H, m), 2.15-2.19 (1H, m), 2.32 (2H, q, J = 7.6 H z), 2.38 (2H, q, J = 7.6 H z), 2.65 (1H, quint, J = 7.6 Hz), 2.88 (1H, d, J = 1.6 Hz), 3.68 (1H, d, J = 12.4 Hz), 3.83 (1H, d, J = 11.9 H z), 4.80 (1H, dd, J = 4.9, 11.3 H z), 5.00 (2H, m), 6.38 (1H, s), 7.40 (1H, dd, J = 4.6, 8.1 Hz), 8.09 (1H, dt J = 1.9, 8.1 Hz), 8.69 (1H, dd, J = 1.6, 4.6 Hz), 9.00 (1H, d, J = 1.6 Hz) |

[Table 19]

**Table 19**

| Compound No. | ¹H-NMR *δ* (ppm) |
|---|---|
| 208 | 0.92 (3H, s), 1.14 (3H, t, J = 7.6 Hz), 1.21 (3H, t, J = 7.6 Hz), 1.26 (1H, s), 1.39-1.47 (1H, m), 1.50 (3H, s), 1.61 (1H, m), 1.68-1.83 (2H, m), 1.86 (3H,s), 1.91-2.05 (2H, m), 2.18-2.23 (1H, m), 2.33 (2H, q, J = 7.6 Hz), 2.43 (2H, dq, J = 1.4, 7.6 Hz), 2.95 (1H, d, J = 2.4 Hz), 3.72 (1H, d, J = 11.9 Hz), 3.82 (1H, d, J = 11.9 Hz), 4.83 (1H, dd, J = 5.1, 11.1 Hz), 5.03-5.06 (1H, m), 5.26 (1H, dd, J = 4.9, 11.1 Hz), 6.40 (1H, s), 7.38 (1H, dd, J = 4.9, 8.4 Hz), 7.76 (2H, d, J = 8.4 Hz), 8.06 (1H, dt, J = 2.2, 8.1 Hz), 8.22 (2H, d, J = 8.4 Hz), 8.66 (1H, dd, J = 1.6, 4.9 Hz), 8.96 (1H, d, J = 2.2 Hz) |
| 211 | 0.90 (3H, s), 1.14 (3H, t, J = 7.6 Hz), 1.15 (3H, t, J = 7.6 Hz), 1.26 (1H, s), 1.29-1.38 (1H, m), 1.41 (3H, s), 1.43-1.71 (5H, m), 1.59 (3H, s), 1.75-1.89 (6H, m), 2.12-2.17 (1H, m), 2.26-2.38 (4H, m), 2.86 (1H, m), 3.45-4.00 (5H, m), 4.82 (1H, dd, J = 5.4, 10.8 Hz), 4.97-5.03 (2H, m), 6.41 (1H, s), 7.40 (1H, dd, J = 4.9, 7.8 Hz), 8.07-8.13 (1H, m), 8.67-8.70 (1H, m), 9.01 (1H, d, J = 2.4 Hz) |
| 212 | 0.92 (3H, s), 1.14 (3H, t, J = 7.6 Hz), 1.20 (3H, t, J = 7.6 Hz), 1.26 (1H, s), 1.38-1.46 (1H, m), 1.50 (3H, s), 1.61 (1H, m), 1.66-1.78 (2H, m), 1.84 (3H, s), 1.87-1.99 (2H, m), 2.12-2.23 (1H, m), 2.31 (2H, q, J = 7.6 Hz), 2.41 (2H, q, J = 7.6 Hz), 2.95 (1H, m), 3.73 (1H, d, J = 11.9 Hz), 3.81 (1 H, d, J = 11.9 Hz), 4.83 (1H, dd, J = 4.9, 11.3 Hz), 5.04 (1H, m), 5.25 (1H, dd, J = 4.9, 11.3 Hz), 6.40 (1H, s), 7.38 (1H, dd, J = 4.6, 7.8 Hz), 7.47 (1H, d, J = 8.1 Hz), 8.06 (1H, dt, J = 1.6, 7.8 Hz), 8.30 (1H, dd, J = 2.4, 8.1 Hz), 8.67 (1H, dd, J = 1.4, 4.6 Hz), 8.97 (1H, d, J = 2.4 Hz), 9.06 (1H, d, J = 2.7 Hz) |
| 213 | 0.90 (3H, s), 0.93 (2H, d, J = 2.7 Hz), 0.96 (2H, d, J = 2.7 Hz), 1.03-1.19 (6H, m), 1.26 (1H, s), 1.32-1.39 (1H, m), 1.45 (3H, s), 1.52 (1H, d, J = 3.8 Hz), 1.61-1.69 (3H, m), 1.71 (3H,s), 1.73-1.94 (2H, m), 2.14-2.19 (1H, m), 2.24-2.40 (4H, m), 2.95 (1H, m), 3.68 (1H, d, J = 11.9 Hz), 3.81 (1H, d, J = 11.9 Hz), 4.79 (1H, dd, J = 5.4, 11.3 Hz), 4.96-5.00 (2H, m), 6.45 (1H, s), 7.40 (1H, dd, J = 4.6, 8.1 Hz), 8.10 (1H, dt, J = 1.9, 8.1 Hz), 8.68 (1H, m), 9.01 (1H, m) |
| 214 | 0.90 (3H, s), 1.13 (3H, t, J = 7.6 Hz), 1.17 (3H, t, J = 7.6 Hz), 1.26 (1H, s), 1.34-1.40 (1H, m), 1.44 (3H, s), 1.54 (1H, d, J = 4.3 Hz), 1.61-1.67 (2H, m), 1.69 (3H,s), 1.72-2.42 (13H, m), 2.91 (1H, m), 3.23 (1H, quint., J = 8.1 Hz), 3.69 (1H, d, J = 11.9 Hz), 3.81 (1H, d, J = 11.9 Hz), 4.80 (1H, dd, J = 4.9, 11.3 Hz), 4.99-5.04 (2H, m), 6.40 (1H, s), 7.39 (1H, dd, J = 4.9, 8.1 Hz), 8.09 (1H, dt, J = 1.6, 8.1 Hz), 8.69 (1H, dd, J = 1.6, 4.6 Hz), 9.01 (1H, d, J = 1.6 Hz) |
| 215 | 0.90 (3H, s), 1.13 (3H, t, J = 7.6 Hz), 1.17 (3H, t, J = 7.6 Hz), 1.26 (1H, s), 1.41-1.46 (1H, m), 1.59 (3H, s), 1.65-1.68 (3H, m), 1.73 (3H, s), 1.84-1.90 (2H, m), 2.18 (1H, m), 2.31 (2H, q, J = 7.6 Hz), 2.38 (2H, q, J = 7.6 Hz), 2.93 (1H, m), 3.69 (1H, d, J = 11.9 Hz), 3.81 (1H, d, J = 11.9 Hz), 4,80(1H, m), 5.01-5.09 (2H, m), 5.92 (1H, dd, J = 1.6, 10.5 Hz), 6.15-6.24 (1H, m), 6.45 (1H, s), 6.45-6.53 (1H, m), 7.40 (1H, dd, J = 4.6, 7.8 Hz), 8.07-8.11 (1H, m), 8.68 (1H, dd, J = 1.9, 4.9 Hz), 9.00 (1H, d, J = 2.2 Hz) |

[Table 20]

**Table20**

| Compound No. | ¹H-NMR *δ* (ppm) |
|---|---|
| 216 | 0.92 (3H, s), 1.14 (3H, t, J = 7.6 Hz), 1.20 (3H, t, J = 7.6 Hz), 1.26 (1H, s), 1.38-1.42 (1H, m), 1.50 (3H, s), 1.64-1.78 (3H, m), 1.85 (3H,s), 1.88-2.05 (2H, m), 2.17-2.23 (1H, m), 2.33 (2H, q, J = 7.6 Hz), 2.42 (2H, dq, J = 1.1, 7.6 Hz), 2.99 (1 H, m), 3.72 (1H, d, J = 12.4 Hz), 3.81 (1H, d, J = 11.5 Hz), 4.83 (1H, dd, J = 4.9, 11.5 Hz), 5.03-5.05 (1H, m), 5.25 (1H, dd, J = 5.4, 11.5 Hz), 6.41 (1H, s), 7.37 (1H, dd, J = 5.2, 8.1 Hz), 7.91 (2H, dd, J = 1.6, 4.6 Hz), 8.07 (1H, dt, J = 1.6, 8.1 Hz), 8.67 (1H, dd, J = 1.9, 4.9 Hz), 8.83 (2H, dd, J = 1.6, 4.3 Hz), 8.97 (1H, d, J = 1.6 Hz) |
| 217 | 0.91 (3H, s), 1.13 (3H, t, J = 7.6 Hz), 1.20 (3H, t, J = 7.6 Hz), 1.26 (1H, s), 1.37-1.46 (1H, m), 1.50 (3H, s), 1.63-1.75 (3H, m), 1.87 (3H, s), 1.83-1.96 (2H, m), 2.13-2.23 (1H, m), 2.32 (2H, q, J = 7.6 Hz), 2.41 (2H, dq, J = 1.4, 7.6 Hz), 2.99 (1H, m), 3.67 (1H, d, J = 11.9 Hz), 3.83 (1H, d, J = 11.9 Hz), 4.83 (1H, dd, J = 5.4, 11.3 Hz), 4.98-5.06 (1H, m), 5.38 (1H, dd, J = 5.4, 10.8 Hz), 6.43 (1H, s), 7.35-7.44 (1H, m), 7.50-7.55 (1H, m), 7.89 (1H, dt, J = 1.6, 7.6 Hz), 8.07 (1H, dt, J = 1.6, 8.1 Hz), 8.18 (1H, d, J = 7.6 Hz), 8.67 (1H, dd, J = 1.6, 4.9 Hz), 8.82-8.84 (1H, m), 8.97 (1H, d, J = 2.4 Hz) |
| 218 | 0.83-1.12 (12H, m), 0.91 (3H, s), 1.26 (1H, s), 1.33-1.41 (1H, m), 1.45 (3H, s), 1.52-1.69 (6H, m), 1.71 (3H, s), 1.81-1.93 (2H, m), 2.14-2.18 (1H, m), 2.92 (1H, m), 3.72 (1H, d, J = 11.9 Hz), 3.82 (1H, d, J = 11.9 Hz), 4.80 (1H, dd, J = 4.9, 11.4 Hz), 4.99-5.04 (2H, m), 6.46 (1H, s), 7.41 (1H, dd, J = 4.9, 8.3 Hz), 8.10 (1H, dt, J = 1.7, 8.3 Hz), 8.69 (1H, dd, J = 1.5, 4.9 Hz), 9.01 (1H, d, J = 1.4 Hz) |
| 219 | 0.90 (3H, s), 1.26 (1H, s), 1.32-1.41 (1H, m), 1.44 (3H, s), 1.51-1.63 (3H, m), 1.69 (3H, s), 1.79-2.04 (8H, m), 2.17-2.40 (14H, m), 2.89 (1H, m), 3.08-3.26 (3H, m), 3.67 (1H, d, J = 11.9 Hz), 3.78 (1H, d, J = 11.9 Hz), 4.79 (1H, dd, J = 5.4, 11.1 Hz), 4.97-5.00 (2H, m), 6.41 (1H, s), 7.41 (1H, dd, J = 4.9, 8.1 Hz), 8.09 (1H, dt, J = 1.9, 8.4 Hz), 8.68 (1H, m), 9.00(1H, m) |
| 220 | 1.17 (3H, s), 1.26 (1H, s), 1.57 (3H, s), 1.65 (1H, m), 1.77-1.82 (2H, m), 1.88 (3H, s), 1.94-2.05 (3H, m), 2.13-2.31 (1H, m), 2.95 (1H, m), 4.16 (2H, s), 5.06 (1H, dd, J = 2.4, 6.5 Hz), 5.17-5.32 (2H, m), 6.42 (1H, s), 7.34-7.64 (10H, m), 8.01-8.12 (7H, m), 8.66 (1H, dd, J = 1.6, 5.1 Hz), 8.97 (1H, d, J = 1.9 Hz) |
| 221 | 0.92 (3H, s), 1.14 (3H, t, J = 7.6 Hz), 1.21 (3H, t, J = 7.6 Hz), 1.26 (1H, s), 1.44 (1H, m), 1.50 (3H, s), 1.57-1.62 (1H, m), 1.67-1.80 (2H, m), 1.85 (3H, s), 1.91-1.95 (2H, m), 2.17-2.24 (1H, m), 2.33 (2H, q, J = 7.6 Hz), 2.42 (2H, q, J = 7.6 Hz), 2.92 (1 H, m), 3.74 (1H, d, J = 11.9 Hz), 3.81 (1H, d, J = 11.9 Hz), 4.84 (1H, dd, J = 4.9, 11.1 Hz), 5.04 (1H, m), 5.27 (1H, dd, J = 4.9, 11.1 Hz), 6.40 (1H, s), 7.38 (1H, dd, J = 4.9, 8.1 Hz), 7.84 (1H, d, J = 8.4 Hz), 8.05-8.08(1H, m), 8.54 (1H, d, J = 8.1 Hz), 8.67 (1H, d, J = 4.6 Hz), 8.96 (1H, d, J = 2.2 Hz), 9.38 (1H, s) |

[Table 21]

**Table 21**

| Compound No. | ¹H-NMR *δ* (ppm) |
|---|---|
| 222 | 0.94 (3H, s), 1.14 (3H, t, J = 7.6 Hz), 1.19 (3H, t, J = 7.6 Hz), 1.26 (1H, s), 1.38-1.47 (1H, m), 1.48 (3H, s), 1.57-1.71 (3H, m), 1.75 (3H, s), 1.83-1.97 (2H, m), 2.10-2.22 (1H, m), 2.33 (2H, q, J = 7.6 Hz), 2.41 (2H, dq, J = 1.6, 7.6 Hz), 2.96 (1H, m), 3.74-3.80 (2H, m), 4.83 (1H, dd, J = 5.7, 11.6 Hz), 5.02-5.03 (1H, m), 5.28 (1H, dd, J = 5.4, 11.6 Hz), 6.41 (1H, s), 7.40 (1H, dd, J = 5.4, 7.6 Hz), 7.69 (1H, d, J = 5.4 Hz), 8.08 (1H, dt, J = 2.2, 8.1 Hz), 8.69 (1H, dd, J = 1.6, 4.9 Hz), 8.97 (1H, d, J = 4.6 Hz), 9.00 (1H, d, J = 2.4 Hz), 9.16 (1H, s) |
| 223 | 0.94 (3H, s), 1.26 (1H, s), 1.37 (1H, m), 1.47 (3H, s), 1.48-1.66 (3H, m), 1.71 (3H, s), 1.75-1.96 (2H, m), 2.17-2.24 (1H, m), 2.96 (1H, m), 3.14-3.35 (6H, m), 3.85 (1H, d, J = 12.2 Hz), 3.93 (1H, d, J = 12.2 Hz), 4.87 (1H, dd, J = 5.7, 10.8 Hz), 4.99-5.08 (2H, m), 6.41 (1H, s), 7.41 (1H, dd, J = 4.6, 8.1 Hz), 8.09 (1H, m), 8.69 (1H, m), 9.02 (1H, m) |
| 224 | 0.91 (3H, s), 1.13 (3H, t, J = 7.3 Hz), 1.17 (3H, t, J = 7.3 Hz), 1.26 (1H, s), 1.40 (1H, m), 1.45 (3H, s), 1.58-1.63 (3H, m), 1.70 (3H, s), 1.73-1.89 (2H, m), 2.10-2.18 (1H, m), 2.32 (2H, q, J = 7.6 Hz), 2.36 (2H, q, J = 7.6 Hz), 2.96 (1H, m), 3.25 (1H, d, J = 9.7 Hz), 3.32 (1 H, d, J = 9.7 Hz), 3.69-3.81 (2H, m), 4.80 (1H, dd, J = 5.4, 11.3 Hz), 5.00-5.08 (2H, m), 6.40 (1H, s), 7.41 (1H, dd, J = 4.9, 8.1 Hz), 8.09 (1H, m), 8.69 (1H, dd, J = 1.4, 5.1 Hz), 9.01 (1H, d, J = 2.4 Hz) |
| 225 | 0.88 (3H, s), 1.13 (3H, t, J = 7.6 Hz), 1.19 (3H, t, J = 7.5 Hz), 1.22 (3H, t, J = 7.6 Hz), 1.24 (3H, s), 1.26 (1H, m), 1.50-1.55 (1H, m), 1.56 (3H, s), 1.55-1.64 (3H, m), 1.70-1.84 (2H, m), 2.31 (2H, dq, J = 1.2, 7.8 Hz), 2.42 (2H, dq, J = 3.4, 13.6 Hz), 2.44 (2H, dq, J = 2.0, 7.5 Hz), 2.79 (1H, dt, J = 1.4, 5.1 Hz), 3.69 (1H, d, J = 11.9 Hz), 3.79 (1H, d, J = 11.9 Hz), 4.79 (1H, dd, J = 4.9, 11.4 Hz), 5.24 (1H, dd, J = 4.9, 11.4 Hz), 6.45 (1H, s), 7.47 (1H, d, J = 8.5 Hz), 8.12 (1H, dd, J = 2.7, 8.5 Hz), 8.83 (1H, d, J = 2.7 Hz) |
| 226 | 0.89 (3H, s), 1.13 (3H, t, J = 7.6 Hz), 1.19 (3H, t, J = 7.6 Hz), 1.10-1.24 (3H, m), 1.26 (1H, s), 1.31-1.39 (1H, m), 1.44 (3H, s), 1.53 (1H, d, J = 3.8 Hz), 1.61-1.67 (2H, m), 1.69 (3H, s), 1.72-1.92 (2H, m), 2.08-2.18 (1H, m), 2.31 (2H, dq, J = 2.7, 7.6 Hz), 2.44 (2H, dq, J = 1.6, 7.6 Hz), 2.26-2.64 (2H, m), 2.85 (1H, s), 3.69 (1H, d, J = 11.9 Hz), 3.80 (1H, d, J = 11.9 Hz), 4.80 (1H, dd, J = 5.4, 11.3 Hz), 4.92-5.10 (2H, m), 6.41 (1H, s), 7.44 (1H, d, J = 8.4 Hz), 8.05 (1H, dd, J = 2.4, 8.4 Hz), 8.78 (1H, d, J = 2.4 Hz) |
| 227 | 0.88 (3H, s), 1.14 (3H, t, J = 7.6 Hz), 1.20 (3H, t, J = 7.6 Hz), 1.23-1.33 (1H, m), 1.43 (1H, m), 1.49 (3H, s), 1.61-1.74 (3H, m), 1.82 (3H, s), 1.87-2.23 (3H, m), 2.33 (2H, q, J = 7.6 Hz), 2.42 (2H, q, J = 7.6 Hz), 2.96 (1H, m), 3.73 (1H, d, J = 12.4 Hz), 3.82 (1H, d, J = 12.4 Hz), 4.83 (1H, dd, J = 5.4, 11.3 Hz), 5.03 (1H, m), 5.26 (1H, dd, J = 5.4, 11.3 Hz), 6.43 (1H, s), 7.39 (1H, dd, J = 4.9, 8.1 Hz), 7.86 (1H, t, J = 5.4 Hz), 8.08 (1H, dt, J = 1.9, 7.8 Hz), 8.60 (1H, d, J = 2.2 Hz), 8.66-8.68 (2H, m), 8.98 (1H, d, J = 2.2 Hz) |

[Table 22]

**Table 22**

| Compound No. | ¹H-NMR *δ* (ppm) |
|---|---|
| 228 | 0.93 (3H, s), 1.14 (3H, t, J = 7.6 Hz), 1.20 (3H, t, J = 7.6 Hz), 1.26 (1H, s), 1.32-1.44 (1H, m), 1.49 (3H, s), 1.61 (1H, d, J = 4.1 Hz), 1.67-1.75 (2H, m), 1.81 (3H,s), 1.79-2.05 (2H, m), 2.13-2.22 (1H, m), 2.33 (2H, q, J = 7.6 Hz), 2.42 (2H, dq, J = 1.4, 7.6 Hz), 2.92 (1H, m), 3.74 (1H, d, J = 11.9 Hz), 3.82 (1H, d, J = 11.9 Hz), 4.84 (1H, dd, J = 5.4, 10.8 Hz), 5.04 (1H, m), 5.27 (1H, dd, J = 5.4, 10.8 Hz), 6.43 (1H, s), 7.40 (1H, dd, J = 4.9, 8.1 Hz), 7.74 (1H, d, J = 5.1 Hz), 8.08 (1H, dt, J = 2.2, 8.1 Hz), 8.65 (1H, d, J = 4.9 Hz), 8.69 (1H, dd, J = 4.1, 7.6 Hz), 8.78 (1H, s), 8.99 (1H, d, J = 1.9 Hz) |
| 229 | 0.92 (3H, s), 1.14 (3H, t, J = 7.6 Hz), 1.19 (3H, t, J = 6.5 Hz), 1.26 (1H, s), 1.34-1.45 (1H, m), 1.49 (3H, s), 1.62 (1H, m), 1.71-1.77 (2H, m), 1.83 (3H, s), 1.88-2.01 (2H, m), 2.14-2.22 (1H, m), 2.33 (2H, q, J = 7.6 Hz), 2.42 (2H, dq, J = 2.2, 7.6 Hz), 2.64 (3H, s), 2.96 (1H, m), 3.72 (1H, d, J = 11.9Hz), 3.84 (1 H, d, J = 11.9 Hz), 4.84 (1H, dd, J = 5.4, 11.3 Hz), 5.04 (1H, m), 5.36 (1H, dd, J = 5.4, 10.8 Hz), 6.42 (1H, s), 7.35-7.42 (2H, m), 7.66 (1H, d, J = 7.8 Hz), 8.08 (1H, dt, J = 1.9, 7.8 Hz), 8.60 (1H, d, J = 4.1 Hz), 8.68 (1H, dd, J = 1.6, 4.9 Hz), 8.98 (1H, d, J = 2.4 Hz), |
| 230 | 0.78 (3H, s), 1.09 (3H, t, J = 7.8 Hz), 1.12 (3H, t, J = 7.8 Hz), 1.26 (1H, s), 1.33 (3H, s), 1.36-1.38 (1H, m), 1.40-1.48 (2H, m), 1.55 (3H, s), 1.59-1.85 (2H, m), 2.09-2.18 (1H, m), 2.32 (4H, q, J = 7.6 Hz), 2.96 (1H, m), 3.40 (1H, d, J = 11.9 Hz), 3.75 (1H, d, J = 11.9 Hz), 4.72 (1 H, dd, J = 4.9, 11.3 Hz), 4.95 (1H, m), 5.17 (1H, dd, J = 5.4, 11.9 Hz), 6.45 (1H, s), 7.40 (1H, dd, J = 4.9, 8.1 Hz), 7.49-7.67 (3H, m), 7.83-7.88 (4H, m), 8.02 (1H, s), 8.07 (1H, dt, J = 2.2, 8.1 Hz), 8.68 (1 H, dd, J = 1.4, 4.6 Hz), 8.95 (1H, dd, J = 1.6, 4.6 Hz), 8.99 (1H, d, J = 1.9 Hz) |
| 231 | 0.91 (3H, s), 1.09 (3H, t, J = 7.6 Hz), 1.14 (3H, t, J = 7.6 Hz), 1.18 (3H, t, J = 7.6 Hz), 1.26 (1H, s), 1.34-1.43 (1H, m), 1.48 (3H, s), 1.63 (1H, m), 1.67-1.75 (2H, m), 1.80 (3H, s), 1.83-2.08 (4H, m), 2.17-2.25 (1H, m), 2.32 (2H, q, J = 7.6 Hz), 2.40 (2H, dq, J = 7.6, 1.9 Hz), 2.96 (1H, m), 3.64 (1H, d, J = 11.9 Hz), 3.87 (1H, d, J = 11.9 Hz), 4.05 (2H, t, J = 6.2 Hz), 4.82 (1H, dd, J = 5.4, 10.8 Hz), 5.04 (1H, m), 5.40 (1H, dd, J = 5.4, 10.8 Hz), 6.47 (1H, s), 7.19-7.44 (3H, m), 8.08 (1H, dt, J = 1.9, 8.1 Hz), 8.32 (1H, dd, J = 1.6, 4.3 Hz), 8.68 (1H, dd, J = 4.6, 1.6 Hz), 8.98 (1H, d, J = 1.6 Hz) |
| 232 | 0.92 (3H, s), 1.14 (3H, t, J = 7.6 Hz), 1.20 (3H, t, J = 7.6 Hz), 1.26 (1H, s), 1.34-1.43 (1H, m), 1.50 (3H, s), 1.61 (1H, m), 1.67-1.78 (2H, m), 1.84 (3H, s), 1.87-1.97 (2H, m), 2.13-2.23 (1H, m), 2.18 (1H,s), 2.32 (2H, q, J = 7.6 Hz), 2.42 (2H, dq, J = 1.4, 7.6 Hz), 3.73 (1H, d, J = 11.9 Hz), 3.80 (1H, d, J = 11.9 Hz), 4.83 (1H, dd, J = 5.4, 11.1 Hz), 5.04 (1H, d, J = 3.8 Hz), 5.25 (1H, dd, J = 5.1, 11.1 Hz), 6.41 (1H, s), 7.06 (1H, dd, J = 3.0, 8.6 Hz), 7.38 (1H, dd, J = 4.9, 8.1 Hz), 8.08 (1H, dt, J = 1.9, 8.1 Hz), 8.43-8.50 (1H, m), 8.67 (1H, dd, J = 1.6, 4.6 Hz), 8.95-8.98 (2H, m) |

[Table 23]

**Table 23**

| Compound No. | ¹H-NMR *δ* (ppm) |
|---|---|
| 233 | 0.91 (3H, s), 1.26 (1H, s), 1.45 (3H, s), 1.70 (3H, s), 1.32-1.97 (29H, m), 2.14-2.19 (1H, m), 2.66-2.90 (3H, m), 3.06 (1H, s), 3.67 (1H, d, J = 11.9 Hz), 3.78 (1H, d, J = 11.9 Hz), 4.78 (1H, dd, J = 5.4, 10.8 Hz), 4.98-5.01 (2H, m), 6.40 (1H, s), 7.42 (1H, dd, J = 4.9, 8.1 Hz), 8.11 (1H, dt, J = 1.6, 8.1 Hz), 8.69 (1H, d, J = 4.6 Hz), 9.01 (1H, s) |
| 234 | 0.91 (3H, s), 1.45 (3H, s), 1.70 (3H, s), 1.10-2.05 (37H, m), 2.14-2.49 (3H, m), 3.04 (1H, s), 3.65 (1H, d, J = 11.3 Hz), 3.77 (1H, d, J = 11.9 Hz), 4.78 (1H, dd, J = 5.4, 10.8 Hz), 4.97-5.01 (2H, m), 6.41 (1H, s), 7.42 (1H, dd, J = 4.9, 8.1 Hz), 8.11 (1H, dd, J = 1.9, 8.1 Hz), 8.69 (1H, d, J = 4.3 Hz), 9.01 (1H, s) |
| 235 | 1.00 (3H, s), 1.25-1.33 (3H, m), 1.48 (3H, s), 1.55 (1H, m), 1.71 (1H, m), 1.75 (3H, s), 1.79-1.98 (2H, m), 2.11-2.21 (1H, m), 3.48 (2H, s), 3.54 (2H, s), 3.60 (2H, s), 3.90 (1H, d, J = 11.9 Hz), 3.99 (1H, d, J = 11.9 Hz), 4.86 (1H, m), 4.98 (1H, m), 5.07-5.12 (1H, m), 6.53 (1H, s), 7.53 (1H, dd, J = 4.9, 8.1 Hz), 8.23 (1H, m), 8.30 (1H, m), 8.70 (1H, m), 9.05 (1H, m) |
| 236 | 0.11-0.27 (8H, m), 0.52-0.65 (8H, m), 0.88 (3H, s), 0.99-1.14 (5H, m), 1.15 (3H, s), 1.25-1.43 (2H, m), 1.61-1.76 (4H, m), 1.72 (3H, s), 2.18-2.54 (9H, m), 3.74 (1H, d, J = 11.9 Hz), 3.83 (1H, d, J = 11.9 Hz), 4.86 (1H, dd, J = 4.6, 11.6 Hz), 5.01-5.12 (2H, m), 6.41 (1H, s), 7.45 (1H, dd, J = 4.9, 7.8 Hz), 8.16 (1H, m), 8.71 (1H, m), 9.02 (1H, s) |
| 237 | 0.14-0.26 (6H, m), 0.52-0.64 (6H, m), 0.92 (3H, s), 0.97-1.16 (4H, m), 1.26-1.38 (1H, m), 1.45 (3H, s), 1.52 (1H, m), 1.63-1.70 (2H, m), 1.70 (3H, s), 1.82-1.91 (2H, m), 2.12-2.41 (7H, m), 2.96 (1H, m), 3.74 (1H, d, J = 11.9 Hz), 3.86 (1H, d, J = 11.9 Hz), 4.84 (1H, dd, J = 4.9, 11.3 Hz), 5.00-5.03 (2H, m), 6.43 (1H, s), 7.42 (1H, dd, J = 4.6, 7.8 Hz), 8.11 (1H, m), 8.70 (1H, d, J = 4.3 Hz), 9.01 (1H, s) |
| 238 | 0.91 (3H, s), 1.26 (1H, s), 1.44 (3H, s), 1.45 (3H, s), 1.46 (3H, s), 1.34-1.53 (7H, m), 1.52 (3H, s), 1.70 (3H, s), 1.81-2.02 (2H, m), 2.15-2.31 (3H, m), 2.96 (1H, s), 3.67 (1H, m), 4.00 (1H, m), 4.85-5.00 (3H, m), 6.46 (1H, s), 7.45 (1H, dd, J = 4.9, 8.1 Hz), 8.13 (1H, m), 8.70 (1H, m), 9.02 (1H, s) |
| 239 | 0.93 (3H, s), 1.14 (3H, t, J = 7.6 Hz), 1.20 (3H, t, J = 7.6 Hz), 1.26 (1H, s), 1.33-1.44 (1H, m), 1.50 (3H, s), 1.61 (1H, m), 1.68-1.77 (2H, m), 1.84 (3H, s), 1.91-1.99 (2H, m), 2.17-2.23 (1H, m), 2.32 (2H, q, J = 7.6 Hz), 2.43 (2H, dq, J = 3.0, 7.6 Hz), 2.69 (3H, s), 2.96 (1H, m), 3.75 (1H, d, J = 12.2 Hz), 3.80 (1H, d, J = 12.2 Hz), 4.48 (1H, dd, J = 5.1, 11.1 Hz), 5.04 (1H, d, J = 4.1 Hz), 5.23 (1H, d, J = 5.4, 10.8 Hz), 6.42 (1H, s), 7.24 (1H, d, J = 5.9 Hz), 7.39 (1H, dd, J = 4.9, 8.1 Hz), 8.08 (1H, d, J = 8.4 Hz), 8.61 (1H, d, J = 5.1 Hz), 8.67 (1H, d, J = 3.5 Hz), 8.98 (1H, s), 9.17 (1H, s) |

[Table 24]

**Table 24**

| Compound No. | ¹H-NMR *δ* (ppm) |
|---|---|
| 240 | 0.93 (3H, s), 1.13 (3H, t, J = 7.9 Hz), 1.19 (3H, t, J = 7.9 Hz), 1.26 (1H, s), 1.39-1.43 (1H, m), 1.49 (3H, s), 1.61 (1H, m), 1.68-1.79 (2H, m), 1.82 (3H, s), 1.88-2.04 (2H, m), 2.17-2.23 (1H, m), 2.32 (2H, q, J = 7.6 Hz), 2.42 (2H, dq, J = 1.9, 7.6 Hz), 2.96 (1H, s), 3.74 (1H, d, J = 11.9 Hz), 3.83 (1H, d, J = 11.9 Hz), 4.83 (1H, dd, J = 1.6, 5.4 Hz), 5.04 (1H, d, J = 4.1 Hz), 5.27 (1H, dd, J = 5.4, 11.6 Hz), 6.43 (1H, s), 7.39 (1H, dd, J = 4.9, 8.1 Hz), 7.47 (1H, d, J = 5.1 Hz), 8.08 (1H, dt, J = 1.9, 8.1 Hz), 8.68 (1H, dd, J = 1.4, 4.6 Hz), 8.64 (1H, d, J = 5.1 Hz), 8.99 (1H, d, J = 1.9 Hz), 9.14 (1H, s) |
| 241 | 0.93 (3H, s), 1.14 (3H, t, J = 7.6 Hz), 1.19 (3H, t, J = 7.6 Hz), 1.26 (1H, s), 1.38-1.43 (1H, m), 1.49 (3H, s), 1.59 (1H, d, J = 4.4 Hz), 1.66-1.73 (2H, m), 1.78 (3H, s), 1.82-2.05 (2H, m), 2.18-2.23 (1H, m), 2.31 (2H, q, J = 7.6 Hz), 2.41 (2H, dq, J = 1.4, 7.6 Hz), 2.96 (1H, s), 3.72 (1H, d, J = 7.6 Hz), 3.81 (1H, d, J = 7.6 Hz), 3.98 (3H, s), 4.84 (1H, dd, J = 5.4, 11.3 Hz), 5.04 (1H, m), 5.24 (1H, dd, J = 4.9, 10.8 Hz), 6.54 (1H, s), 7.39 (1H, dd, J = 4.9, 8.1 Hz), 7.53 (1H, d, J = 4.9 Hz), 8.08 (1H, dt, J = 1.9, 8.1 Hz), 8.68 (1H, d, J = 4.1 Hz), 8.88 (1H, d, J = 4.9 Hz), 9.00 (1H, s), 9.17 (1H, s) |
| 242 | 0.95 (3H, s), 1.15 (3H, t, J = 7.6 Hz), 1.20 (3H, t, J = 7.6 Hz), 1.26 (1H, s), 1.38-1.44 (1H, m), 1.49 (3H, s), 1.61 (1H, d, J = 4.1 Hz), 1.68-1.72 (2H, m), 1.76 (3H, s), 1.82-2.06 (2H, m), 2.18-2.23 (1H, m), 2.34 (2H, q, J = 7.6 Hz), 2.43 (2H, dq, J = 2.2, 7.6 Hz), 2.96 (1H, s), 3.78 (1H, d, J = 12.2 Hz), 3.83 (1H, d, J = 12.2 Hz), 4.84 (1H, dd, J = 5.4, 11.3 Hz), 5.04 (1H, d, J = 4.1 Hz), 5.2-5.34 (1H, m), 6.40 (1H, s), 7.40 (1H, dd, J = 4.9, 8.1 Hz), 7.76 (1H, d, J = 5.4 Hz), 8.02-8.11 (2H, m), 8.69 (1H, d, J = 4.3 Hz), 8.74 (1H, s), 9.00 (1H, s) |
| 243 | 0.93 (3H, s), 1.14 (3H, t, J = 7.6 Hz), 1.20 (3H, t, J = 7.6 Hz), 1.26 (1H, s), 1.39-1.44 (1H, m), 1.50 (3H, s), 1.62 (1H, m), 1.68-1.75 (2H, m), 1.84 (3H, s), 1.93-1.96 (2H, m), 2.14-2.23 (1H, m), 2.33 (2H, q, J = 7.6 Hz), 2.42 (2H, dq, J = 2.4, 7.6 Hz), 2.96 (1H, s), 3.72 (1H, d, J = 11.9 Hz), 3.83 (1H, d, J = 11.9 Hz), 4.83 (1H, dd, J = 1.6, 5.4 Hz), 5.04 (1H, m), 5.36 (1H, dd, J = 4.9, 11.3 Hz), 6.46 (1H, s), 7.38 (1H, dd, J = 5.4, 7.6 Hz), 7.68-7.78 (2H, m), 7.83-7.88 (1H, m), 8.07 (1H, dt, J = 1.9, 8.1 Hz), 8.19-8.23 (1H, m), 8.67 (1H, dd, J = 1.6, 4.9 Hz), 8.98 (1H, d, J = 2.2 Hz) |
| 244 | 0.93 (3H, s), 1.14 (3H, t, J = 7.6 Hz), 1.19 (3H, t, J = 7.6 Hz), 1.26 (1H, s), 1.34-1.43 (1H, m), 1.48 (3H, s), 1.60 (1H, d, J = 4.1 Hz), 1.66-2.02 (4H, m), 1.73 (3H, s), 2.11-2.23 (1H, m), 2.33 (2H, q, J = 7.6 Hz), 2.41 (2H, dq, J = 2.2, 7.6 Hz), 2.90 (1H, s), 3.74 (1H, d, J = 11.9 Hz), 5.83 (1H, d, J = 11.9 Hz), 4.82 (1H, dd, J = 4.9, 11.1 Hz), 5.03 (1H, m), 5.27 (1H, dd, J = 5.1, 11.6 Hz), 6.43 (1 H, s), 7.41 (1H, dd, J = 4.9, 8.1 Hz), 7.65-7.70 (2H, m), 7.78-7.86 (2H, m), 8.09 (1H, dt, J = 1.9, 8.1 Hz), 8.69 (1H, d, J = 3.8 Hz), 9.00 (1H, s) |

[Table 25]

**Table 25**

| Compound No. | ¹H-NMR *δ* (ppm) |
|---|---|
| 245 | 0.92 (3H, s), 1.14 (3H, t, J = 7.5 Hz), 1.20 (3H, t, J = 7.5 Hz), 1.26 (1H, s), 1.39-1.46 (1H, m), 1.49 (3H, s), 1.62 (1H, d, J= 4.1 Hz), 1.83 (3H, s), 1.66-2.02 (4H, m), 2.11-2.23 (1H, m), 2.33 (2H, dq, J = 1.2, 7.6 Hz), 2.42 (2H, dq, J = 3.2, 7.6 Hz), 2.96 (1H, m), 3.70 (1H, d, J = 12.0 Hz), 3.85 (1H, d, J = 12.0 Hz), 4.83 (1H, dd, J = 4.9, 11.7 Hz), 5.04 (1H, m), 5.27 (1H, dd, J = 5.1, 11.9 Hz), 6.45 (1H, s), 7.18 (1H, dd, J = 8.5, 10.9 Hz), 7.27 (1H, m), 7.38 (1H, dd, J = 4.8, 8.1 Hz), 7.55-7.61 (1H, m), 8.03 (1H, dt, J = 1.7, 7.3 Hz), 8.08 (1H, dt, J = 1.7, 8.3 Hz), 8.67 (1H, d, J = 3.9 Hz), 8.98 (1H, s) |
| 246 | 0.93 (3H, s), 1.14 (3H, t, J = 7.5 Hz), 1.19 (3H, t, J = 7.5 Hz), 1.26 (1H, s), 1.32-1.42 (1H, m), 1.45 (3H, s), 1.59 (1H, d, J = 3.0 Hz), 1.66 (3H, s), 1.69-1.92 (4H, m), 2.02-2.21 (1H, m), 2.33 (2H, dq, J = 1.1, 5.1 Hz), 2.42 (2H, dq, J = 2.2, 5.1 Hz), 2.96 (1H, m), 3.76 (1H, d, J = 11.9 Hz), 3.84 (1H, d, J = 12.0 Hz), 4.83 (1H, dd, J = 4.9, 11.7 Hz), 5.03 (1H, d, J = 4.2 Hz), 5.19 (1H, dd, J = 5.4, 11.7 Hz), 6.60 (1H, s), 7.42 (1H, dd, J = 4.6, 8.1 Hz), 7.66-7.76 (2H, m), 7.84 (1H, dd, J = 1.5, 7.5 Hz), 7.93 (1H, dd, J = 1.5, 7.8 Hz), 8.11 (1H, dt, J = 2.1, 8.1 Hz), 8.69 (1H, d, J = 4.6 Hz), 9.03 (1H, s) |
| 247 | 0.93 (3H, s), 1.14 (3H, t, J = 7.6 Hz), 1.20 (3H, t, J = 7.6 Hz), 1.26 (1H, s), 1.42-1.46 (1H, m), 1.49 (3H, s), 1.61 (1H, d, J = 3.0 Hz), 1.68-1.79 (2H, m), 1.82 (3H, s), 1.86-2.02 (2H, m), 2.16-2.22 (1H, m), 2.33 (2H, dq, J = 1.1, 5.1 Hz), 2.42 (2H, dq, J = 2.4, 5.1 Hz), 2.96 (1H, m), 3.74 (1H, d, J = 12.0 Hz), 3.82 (1H, d, J = 12.0 Hz), 4.83 (1H, dd, J = 4.9, 11.7 Hz), 5.04 (1H, m), 5.27 (1H, dd, J = 5.1, 11.7 Hz), 6.44 (1H, s), 7.40 (1H, dd, J = 4.6, 7.8 Hz), 7.72 (1H, dd, J = 1.7, 8.3 Hz), 8.08 (1H, dt, J = 2.2, 8.5 Hz), 8.26 (1H, dd, J = 1.9, 7.8 Hz), 8.58 (1H, dd, J = 1.9, 4.9 Hz), 8.68 (1H, d, J = 3.6 Hz), 9.03 (1H, d, J = 1.7 Hz) |
| 248 | 0.93 (3H, s), 1.16 (3H, t, J = 7.6 Hz), 1.22 (3H, t, J = 7.6 Hz), 1.26 (1H, s), 1.42-1.46 (1H, m), 1.49 (3H, s), 1.61 (1H, d, J = 3.0 Hz), 1.68-1.78 (2H, m), 1.82 (3H, s), 1.86-2.01 (2H, m), 2.17-2.22 (1H, m), 2.33 (2H, dq, J = 1.1, 5.1 Hz), 2.42 (2H, dq, J = 2.4, 5.1 Hz), 2.62 (3H, s), 2.98 (1 H, m), 3.73 (1H, d, J = 12.0 Hz), 3.84 (1H, d, J = 11.9 Hz), 4.83 (1H, dd, J = 4.8, 11.5 Hz), 5.04 (1H, d, J = 3.4 Hz), 5.25 (1H, dd, J = 5.1, 11.4 Hz), 6.44 (1H, s), 7.22 (1H, d, J = 7.8 Hz), 7.40 (1H, dd, J = 4.9, 8.0 Hz), 8.08 (1H, dt, J = 2.2, 8.0 Hz), 8.18 (1H, d, J = 7.8 Hz), 8.69 (1H, d, J = 3.7 Hz), 8.99 (1H, d, J = 1.7 Hz) |
| 249 | 0.91 (3H, s), 1.14 (3H, t, J = 7.8 Hz), 1.15 (3H, t, J = 7.8 Hz), 1.26 (1H, s), 1.29-1.39 (1H, m), 1.42 (3H, s), 1.45 (1H, m), 1.57-1.64 (2H, m), 1.66 (3H, s), 1.81-1.88 (2H, m), 2.14-2.18 (1H, m), 2.33 (2H, q, J = 7.8 Hz), 2.35 (2H, q, J = 7.8 Hz), 2.84 (1H, m), 3.46 (3H, s), 3.68 (1H, d, J = 11.7 Hz), 3.93 (1H, d, J = 11.9 Hz), 4.73-4.87 (4H, m), 4.95-5.00 (1H, m), 6.43 (1H, s), 7.42 (1H, dd, J = 4.8, 8.0 Hz), 8.12 (1H, m), 8.69 (1H, m), 9.01 (1H, d, J = 2.2Hz) |
| 250 | 0.92 (3H, s),1.26 (1H, s), 1.34-1.55 (3H, m), 1.46 (3H, s), 1.71 (3H, s), 1.66-1.92 (6H, m), 2.01-2.18 (4H, m), 2.38-2.57 (3H, m), 3.66-3.78 (1H, m), 3.95-4.13 (1H, m), 4.73-4.84 (1H, m), 4.89-4.95 (1H, m), 4.99-5.10 (1H, m), 6,45 (1H, s), 7.43 (1H, dd, J = 4.9, 8.3 Hz), 8.11 (1H, m), 8.70 (1H, d, J = 4.9 Hz), 9.02 (1H, s) |

[Table 26]

**Table 26**

| Compound No. | ¹H-NMR *δ* (ppm) | |
|---|---|---|
| 251 | 0.93 (3H, s), 1.14 (3H, t, J = 7.5 Hz), 1.17 (3H, t, J = 7.5 Hz), 1.26 (1H, s), 1.36 (9H, s), 1.42 (1H, m), 1.47 (3H, s), 1.62-1.70 (3H, m), 1.75 (3H, s), 1.80-1.95 (2H, m), 2.07-2.21 (1H, m), 2.32 (2H, dq, J = 1.5, 7.5 Hz), 2.40 (2H, dq, J = 3.9, 7.6 Hz), 2.96 (1H, m), 3.69 (1H, d, J = 11.9 Hz), 3.87 (1 H, d, J = 11.9 Hz), 4.83 (1H, dd, J = 4.9, 11.7 Hz), 5.04 (1H, m), 5.36 (1H, dd, J = 5.1, 11.7 Hz), 6.53 (1H, s), 7.39-7.43 (1H, m), 7.98 (1H, dd, J = 1.7, 8.0 Hz), 8.02 (1H, s), 8.10 (1H, dt, J = 1.7, 8.0 Hz), 8.65 (1H, dd, J = 1.5, 4.7 Hz), 8.69 (1H, d, J = 3.7 Hz), 8.99 (1H, s) | |
| 252 | 0.92 (3H, s), 1.14 (3H, t, J = 7.5 Hz), 1.19 (3H, t, J = 7.5 Hz), 1.26 (1H, s), 1.42-1.45 (1H, m), 1.49 (3H, s), 1.62-1.73 (3H, m), 1.82 (3H, s), 1.84-2.00 (2H, m), 2.18-2.22 (1H, m), 2.32 (2H, dq, J = 1.5, 7.5 Hz), 2.41 (2H, dq, J = 2.5, 7.5 Hz), 2.96 (1H, m), 3.68 (1H, d, J = 11.9 Hz), 3.85 (1H, d, J = 11.9 Hz), 4.82 (1H, dd, J = 4.9, 11.7 Hz), 5.04 (1H, m), 5.37 (1H, dd, J = 4.8, 11.7 Hz), 6.44 (1H, s), 7.36-7.41 (2H, m), 8.08 (1 H, dt, J = 1.7, 8.0 Hz), 8.53 (1H, d, J = 2.0 Hz), 8.68 (1H, dd, J = 0.7, 4.9 Hz), 8.98 (1H, d, J = 2.6 Hz) | |
| 253 | 0.92 (3H, s), 1.14 (3H, t, J = 7.5 Hz), 1.20 (3H, t, J = 7.5 Hz), 1.26 (1 H, s), 1.40-1.47 (1H, m), 1.51 (3H, s), 1.64 (1H, d, J = 2.4 Hz), 1.73 (2H, m), 1.87 (3H, s), 1.85-2.00 (2H, m), 2.18-2.23 (1H, m), 2.32 (2H, q, J = 7.6 Hz), 2.42 (2H, dq, J = 1.5, 7.6 Hz), 2.96 (1H, m), 3.71 (1H, d, J = 12.0 Hz), 3.83 (1H, d, J = 11.9 Hz), 4.84 (1H, dd, J = 4.9, 11.7 Hz), 5.05 (1H, m), 5.39 (1H, dd, J = 5.2, 11.6 Hz), 6.42 (1H, s), 7.39 (1H, dd, J = 4.9, 8.1 Hz), 8.02 (1H, s), 8.07 (1H, m), 8.68 (1H, d, J = 4.4 Hz), 8.80-8.83 (1H, m), 8.97 (1H, m), 9.38 (1H, m) | |
| 254 | 0.91 (3H, s), 1.14 (3H, t, J = 7.6 Hz), 1.19 (3H, t, J = 7.6 Hz), 1.26 (1H, s), 1.39-1.46 (1H, m), 1.49 (3H, s), 1.63 (1H, d, J = 2.7 Hz), 1.70-1.73 (2H, m), 1.85 (3H, s), 1.88-2.01 (2H, m), 2.18-2.22 (1H, m), 2.32 (2H, q, J = 7.5 Hz), 2.41 (2H, dq, J = 2.2, 7.6 Hz), 2.97 (1H, m), 3.68 (1H, d, J = 11.7 Hz), 3.83 (1H, d, J = 11.9 Hz), 4.83 (1H, dd, J = 4.9, 11.7 Hz), 5.04 (1H, m), 5.34 (1H, dd, J = 5.4, 11.5 Hz), 6.44 (1H, s), 7.39 (1 H, dd, J = 4.9, 8.0 Hz), 8.07 (1H, dt, J = 1.9, 6.3 Hz), 8.32 (1H, d, J = 2.0 Hz), 8.67 (1H, d, J = 4.1 Hz), 8.92 (1H, d, J = 2.0 Hz), 8.98 (1H, s) | |
| 255 | 0.92 (3H, s), 1.14 (3H, t, J = 7.5 Hz), 1.19 (3H, t, J = 7.5 Hz), 1.26 (1H, s), 1.38-1.45 (1H, m), 1.49 (3H, s), 1.60 (1H, d, J = 3.0 Hz), 1.68-1.70 (2H, m), 1.83 (3H, s), 1.75-1.98 (2H, m), 2.17-2.21 (1H, m), 2.33 (2H, dq, J = 1.7, 7.5 Hz), 2.41 (2H, dq, J = 2.2, 7.5 Hz), 2.97 (1H, m), 3.67 (1H, d, J = 12.0 Hz), 3.87 (1H, d, J = 11.9 Hz), 4.81 (1H, dd, J = 4.9, 11.7 Hz), 5.03 (1H, m), 5.23 (1H, dd, J = 5.1, 11.5 Hz), 6.46 (1H, s), 7.07 (1H, d, J = 5.2 Hz), 7.39 (1H, dd, J = 4.9, 8.1 Hz), 7.54 (1H, d, J = 5.3 Hz), 8.08 (1H, dt, J = 2.2, 8.1 Hz), 8.67 (1H, dd, J = 1.4, 4.9 Hz), 8.99 (1H, d, J = 2.2 Hz) | |

[Table 27]

**Table 27**

| Compound No. | ¹H-NMR *δ* (ppm) |
|---|---|
| 256 | 0.92 (3H, s), 1.12 (3H, t, J = 7.8 Hz), 1.15 (3H, t, J = 7.7 Hz), 1.26 (1H, s), 1.39-1.47 (1H, m), 1.50 (3H, s), 1.61 (1H, d, J = 2.4 Hz), 1.69-1.81 (2H, m), 1.85 (3H, s), 1.90-1.99 (2H, m), 2.18-2.21 (1H, m), 2.33 (2H, dq, J = 1.2, 7.7 Hz), 2.41 (2H, dq, J = 2.7, 7.6 Hz), 2.66 (3H, s), 2.96 (1H, m), 3.72 (1H, d, J = 11.7 Hz), 3.83 (1H, d, J = 12.0 Hz), 4.83 (1H, dd, J = 4.9, 11.4 Hz), 5.04 (1H, m), 5.25 (1H, dd, J = 5.3, 11.7 Hz), 6.41 (1H, s), 7.30 (1H, d, J = 8.0 Hz), 7.38 (1H, dd, J = 4.9, 8.1 Hz), 8.07 (1H, dt, J = 2.2, 8.1 Hz), 8.24 (1H, dd, J = 2.2, 8.0 Hz), 8.67 (1H, dd, J = 1.5, 4.9 Hz), 8.97 (1 H, d, J = 2.2 Hz), 9.18 (1H, d, J = 2.2 Hz) |
| 257 | 0.91 (3H, s), 1.14 (3H, t, J = 7.5 Hz), 1.19 (3H, t, J = 7.5 Hz), 1.26 (1H, s), 1.38-1.46 (1H, m), 1.50 (3H, s), 1.63 (1H, d, J = 2.4 Hz), 1.70-1.73 (2H, m), 1.86 (3H, s), 1.83-1.98 (2H, m), 2.18-2.22 (1H, m), 2.32 (2H, dq, J = 1.5, 7.7 Hz), 2.41 (2H, dq, J = 2.2, 7.7 Hz), 2.96 (1H, d, J = 1.9 Hz), 3.68 (1H, d, J = 11.9 Hz), 3.84 (1H, d, J = 12.0 Hz), 4.83 (1H, dd, J = 4.9, 11.7 Hz), 5.05 (1H, m), 5.32 (1H, dd, J = 5.3, 11.7 Hz), 6.43 (1H, s), 7.39 (1H, dd, J = 4.9, 8.0 Hz), 7.56 (1H, d, J = 8.1 Hz), 7.85 (1H, t, J = 7.8 Hz), 8.07 (2H, m), 8.67 (1H, dd, J = 1.7, 4.9 Hz), 8.98 (1H, d, J = 2.0 Hz) |
| 258 | 0.91 (3H, s), 1.14 (3H, t, J = 7.5 Hz), 1.19 (3H, t, J = 7.5 Hz), 1.26 (1 H, s), 1.38-1.46 (1H, m), 1.50 (3H, s), 1.62 (1H, d, J = 2.4 Hz), 1.69-1.72 (2H, m), 1.86 (3H, s), 1.80-1.96 (2H, m), 2.18-2.22 (1H, m), 2.32 (2H, q, J = 7.5 Hz), 2.41 (2H, dq, J = 2.2, 7.5 Hz), 2.93 (1H, d, J = 1.9 Hz), 3.68 (1H, d, J = 11.9 Hz), 3.83 (1H, d, J = 12.0 Hz), 4.83 (1H, dd, J = 4.9, 11.4 Hz), 5.04 (1H, m), 5.33 (1H, dd, J = 5.3, 11.5 Hz), 6.42 (1H, s), 7.20 (1H, dd, J = 2.9, 8.0 Hz), 7.38 (1H, dd, J = 4.9, 8.3 Hz), 8.00 (1H, q, J = 7.8 Hz), 8.08 (2H, m), 8.67 (1H, dd, J = 1.4, 4.6 Hz), 8.97 (1H, d, J = 2.2 Hz) |
| 259 | 0.93 (3H, s), 1.14 (3H, t, J = 7.5 Hz), 1.21 (3H, t, J = 7.5 Hz), 1.26 (1H, s), 1.40-1.47 (1H, m), 1.51 (3H, s), 1.61 (1H, d, J = 3.0 Hz), 1.70-1.83 (2H, m), 1.86 (3H, s), 1.92-1.98 (2H, m), 2.17-2.22 (1H, m), 2.32 (2H, q, J = 7.3 Hz), 2.43 (2H, dq, J = 1.4, 5.3 Hz), 2.97 (1H, d, J = 2.0 Hz), 3.74 (1H, d, J = 11.7 Hz), 3.83 (1H, d, J = 11.7 Hz), 4.13 (3H, s), 4.84 (1H, dd, J = 4.9, 11.4 Hz), 5.05 (1H, m), 5.24 (1H, dd, J = 5.3, 11.7 Hz), 6.43 (1 H, s), 7.16-7.20 (1H, m), 7.35-7.44 (4H, m), 7.70 (1H, d, J = 8.1 Hz), 8.05 (1H, dt, J = 1.7, 8.3 Hz), 8.66 (1H, dd, J = 1.5, 4.9 Hz), 8.96 (1H, d, J = 2.2 Hz) |
| 260 | 0.93 (3H, s), 1.14 (3H, t, J = 7.5 Hz), 1.19 (3H, t, J = 7.5 Hz), 1.26 (1H, s), 1.40-1.46 (1H, m), 1.48 (3H, s), 1.63 (1H, d, J = 3.0 Hz), 1.71-1.74 (2H, m), 1.80 (3H, s), 1.83-1.95 (1H, m), 2.02-2.06 (1H, m), 2.18-2.22 (1H, m), 2.32 (2H, dq, J = 1.7, 7.6 Hz), 2.41 (2H, dq, J = 3.4, 7.5 Hz), 2.96 (1H, m), 3.70 (1H, d, J = 12.0 Hz), 3.87 (1H, d, J = 11.9 Hz), 4.83 (1H, dd, J = 4.8, 11.5 Hz), 5.05 (1H, m), 5.37 (1H, dd, J = 4.9, 11.7 Hz), 6.46(1H, s), 7.39-7.45 (2H, m), 7.87 (1H, dd, J = 1.5, 8.3 Hz), 8.08 (1H, dt, J = 1.5, 8.3 Hz), 8.64 (1H, dd, J = 1.2, 4.6 Hz), 8.69 (1H, d, J = 4.9 Hz), 8.97 (1H, d, J = 2.2 Hz) |
| 261 | 0.85-1.06 (8H, m), 0.92 (3H, s), 1.26 (1H, s), 1.30-1.40 (1H, m), 1.42 (3H, s), 1.45-1.63 (5H, m), 1.67 (3H, s), 1.81-1.92 (2H, m), 2.14-2.25 (2H, m), 2.88 (1H, d, J = 1.4 Hz), 3.75 (1H, d, J = 11.9 Hz), 3.86 (1H, d, J = 11.6 Hz), 3.78-3.82 (1H, m), 4.82 (1H, dd, J = 5.1, 11.4 Hz), 5.00 (1H, m), 6.52 (1H, s), 7.42 (1H, dd, J = 4.9, 8.0 Hz), 8.11 (1H, dt, J = 1.7, 8.0 Hz), 8.69 (1H, dd, J = 1.5, 4.9 Hz), 9.01 (1H, d, J = 1.9 Hz) |

[Table 28]

**Table 28**

| Compound No. | ¹H-NMR δ (ppm) |
|---|---|
| 262 | 0.92 (3H, s), 1.14 (3H, t, J = 7.5 Hz), 1.20 (3H, t, J = 7.5 Hz), 1.26 (1H, s), 1.39-1.47 (1H, m), 1.49 (3H, s), 1.61 (1H, d, J = 2.7 Hz), 1.66-1.71 (2H, m), 1.84 (3H, s), 1.76-1.99 (2H, m), 2.18-2.22 (1H, m), 2.32 (2H, dq, J = 1.0, 7.5 Hz), 2.42 (2H, dq, J = 2.7, 7.5 Hz), 2.96 (1H, m), 3.73 (1H, d, J = 11.9 Hz), 3.82 (1H, d, J = 11.9 Hz), 4.83 (1H, dd, J = 4.9, 11.7 Hz), 5.04 (1H, m), 5.26 (1H, dd, J = 5.1, 11.7 Hz), 6.44 (1H, s), 7.35-7.41 (2H, m), 8.07 (1H, dt, J = 1.7, 8.0 Hz), 8.44-8.50 (2H, m), 8.67 (1H, d, J = 4.9 Hz), 8.98 (1H, d, J = 1.7 Hz) |
| 263 | 0.92 (3H, s), 1.12 (3H, t, J = 7.5 Hz), 1.20 (3H, t, J = 7.5 Hz), 1.26 (1H, s), 1.30-1.47 (1H, m), 1.50 (3H, s), 1.62 (1H, d, J = 2.4 Hz), 1.69-1.71 (2H, m), 1.85 (3H, s), 1.75-1.97 (2H, m), 2.18-2.22 (1H, m), 2.33 (2H, dq, J = 0.9, 7.6 Hz), 2.42 (2H, dq, J = 2.4, 7.6 Hz), 2.98 (1H, m), 3.73 (1H, d, J = 11.6 Hz), 3.81 (1H, d, J = 11.9 Hz), 4.84 (1H, dd, J = 4.9, 11.7 Hz), 5.05 (1H, m), 5.26 (1H, dd, J = 5.1, 11.5 Hz), 6.40 (1H, s), 7.38 (1H, dd, J = 4.9, 8.0 Hz), 7.80 (2H, d, J = 8.8 Hz), 8.06 (1H, dt, J = 1.7, 8.0 Hz), 8.21 (2H, d, J = 8.8 Hz), 8.67 (1H, dd, J = 1.5, 4.9 Hz), 8.96 (1H, d, J = 1.7 Hz) |
| 264 | 0.92 (3H, s), 1.14 (3H, t, J = 7.5 Hz), 1.20 (3H, t, J = 7.5 Hz), 1.26 (1H, s), 1.39-1.47 (1H, m), 1.51 (3H, s), 1.62 (1H, d, J = 2.4 Hz), 1.68-1.82 (2H, m), 1.86 (3H, s), 1.93-2.01 (2H, m), 2.19-2.23 (1H, m), 2.32 (2H, dq, J = 1.0, 7.6 Hz), 2.42 (2H, dq, J = 2.4, 7.5 Hz), 2.97 (1H, m), 3.73 (1H, d, J = 11.9 Hz), 3.80 (1H, d, J = 11.9 Hz), 4.84 (1H, dd, J = 4.9, 11.7 Hz), 5.05 (1H, m), 5.26 (1H, dd, J = 5.1, 11.5 Hz), 6.41 (1H, s), 7.38 (1H, dd, J = 4.1, 8.0 Hz), 7.65 (1H, m), 7.90 (1H, dt, J = 1.5, 7.8 Hz), 8.07 (1H, dt, J = 2.2, 8.0 Hz), 8.34 (1H, dt, J = 1.5, 7.8 Hz), 8.38 (1H, t, J = 1.5 Hz), 8.67 (1H, dd, J = 1.5, 4.9 Hz), 8.96 (1H, d, J = 2.4 Hz) |
| 265 | 0.92 (3H, s), 1.14 (3H, t, J = 7.5 Hz), 1.21 (3H, t, J = 7.5 Hz), 1.26 (1H, s), 1.39-1.48 (1H, m), 1.51 (3H, s), 1.63 (1H, d, J = 2.7 Hz), 1.63-1.83 (2H, m), 1.86 (3H, s), 1.90-1.98 (2H, m), 2.18-2.23 (1H, m), 2.33 (2H, q, J = 7.5 Hz), 2.43 (2H, dq, J = 2.5, 7.6 Hz), 2.97 (1H, m), 3.72 (1H, d, J = 11.9 Hz), 3.82 (1H, d, J = 12.0 Hz), 4.84 (1H, dd, J = 4.9, 11.4 Hz), 5.05 (1H, d, J = 4.1 Hz), 5.28 (1H, dd, J = 5.1, 11.5 Hz), 6.42 (1H, s), 7.38 (1H, dd, J = 4.9, 8.0 Hz), 7.65 (1H, t, J = 7.8 Hz), 7.88 (1H, d, J = 7.8 Hz), 8.06 (1H, dt, J = 1.8, 8.0 Hz), 8.30 (1H, d, J = 8.1 Hz), 8.36 (1H, s), 8.67 (1H, dd, J = 1.5, 4.9 Hz), 8.97 (1H, d, J = 2.2 Hz) |
| 266 | 0.89 (3H, s), 1.13 (3H, t, J = 7.6 Hz), 1.14 (3H, t, J = 7.6 Hz), 1.26 (1H, s), 1.33-1.37 (1H, m), 1.42 (3H, s), 1.46-1.55 (1H, m), 1.58 (3H, s), 1.60-1.70 (2H, m), 1.78-1.91 (2H, m), 2.13-2.17 (1H, m), 2.32 (2H, dq, J = 1.7, 7.3 Hz), 2.35 (2H, q, J = 7.3 Hz), 2.89 (1H, m), 3.66 (1H, d, J = 11.4 Hz), 3.81 (1H, d, J = 12.0 Hz), 3.96 (2H, s), 4.76-4.82 (1H, m), 4.98-5.06 (2H, m), 6.38 (1H, s), 7.17-7.25 (1H, m), 7.36-7.46 (2H, m), 7.69-7.73 (1H, m), 8.08-8.12 (1H, m), 8.60 (1H, dt, J = 1.0, 4.9 Hz), 8.70 (1H, dd, J = 1.7, 4.9 Hz), 9.00 (1H, d, J = 1.4 Hz) |

[Table 29]

**Table 29**

| Compound No. | ¹H-NMR *δ* (ppm) |
|---|---|
| 267 | 0.89 (3H, s), 1.13 (3H, t, J = 7.6 Hz), 1.15 (3H, t, J = 7.6 Hz), 1.26 (1H, s), 1.43 (3H, s), 1.50 (3H, d, J = 3.0 Hz), 1.61 (3H, s), 1.58-1.70 (2H, m), 1.75-1.93 (2H, m), 2.14-2.18 (1H, m), 2.32 (2H, q, J = 7.6 Hz), 2.36 (2H, q, J =7.6 Hz), 2.90 (1H, d, J = 1.9 Hz), 3.70 (1H, d, J = 12.0 Hz), 3.74 (2H, s), 3.77 (1H, d, J = 11.9 Hz), 4.79 (1H, dd, J = 4.9, 11.4 Hz), 4.96-5.00 (2H, m), 6.37 (1H, s), 7.32 (1H, dd, J = 4.8, 7.6 Hz), 7.42 (1H, dd, J = 4.9, 8.1 Hz), 7.71 (1H, d, J = 7.8 Hz), 8.12 (1H, dt, J = 1.9, 8.1 Hz), 8.57 (1H, dd, J = 1.6, 4.8 Hz), 8.65 (1H, d, J = 1.9 Hz), 8.70 (1H, dd, J = 1.6, 4.7 Hz), 9.04 (1H, d, J = 4.2 Hz) |
| 269 | 0.85-1.11 (8H, m), 0.93 (3H, s), 1.26 (1H, s), 1.39-1.47 (1H, m), 1.50 (3H, s), 1.55-1.68 (5H, m), 1.87 (3H, s), 1.83-2.02 (2H, m), 2.17-2.22 (1H, m), 2.96 (1H, s), 3.79 (1H, d, J = 12.2 Hz), 3.83 (1H, d, J = 12.1 Hz), 4.85 (1H, dd, J = 4.9, 11.5 Hz), 5.04 (1H, m), 5.38 (1H, dd, J = 5.12, 11.6 Hz), 6.46 (1H, s), 7.38 (1H, dd, J = 4.8, 8.2 Hz), 7.69-7.80 (2H, m), 7.87 (1H, m), 8.08 (1H, dt, J = 2.2, 8.0 Hz), 8.22 (1H, dd, J = 1.7, 7.5 Hz), 8.67 (1H, dd, J = 1.5, 4.9 Hz), 8.98 (1H, d, J = 2.4 Hz) |
| 270 | 0.86-1.10 (8H, m), 0.94 (3H, s), 1.26 (1H, s), 1.38-1.46 (1H, m), 1.49 (3H, s), 1.57-1.69 (5H, m), 1.75 (3H, s), 1.78-2.05 (2H, m), 2.18-2.21 (1H, m), 2.93 (1H, m), 3.80 (1H, d, J = 11.9 Hz), 3.84 (1H, d, J = 11.9 Hz), 4.84 (1H, dd, J = 5.0, 11.6 Hz), 5.04 (1H, m), 5.31 (1H, dd, J = 5.0, 11.8 Hz), 6.42 (1H, s), 7.40 (1H, dd, J = 4.9, 8.3 Hz), 7.70 (1H, d, J = 5.3 Hz), 8.09 (1H, dt, J = 1.7, 8.1 Hz), 8.69 (1H, dd, J = 1.6, 4.7 Hz), 8.97 (1H, d, J = 5.1 Hz), 9.00 (1H, d, J = 2.2 Hz), 9.17 (1H, s) |
| 271 | 0.85-1.08 (8H, m), 0.92 (3H, s), 1.26 (1H, s), 1.38-1.46 (1H, m), 1.48 (3H, s), 1.56-1.68 (5H, m), 1.79 (3H, s), 1.83-2.08 (2H, m), 2.18-2.21 (1H, m), 2.95 (1H, m), 3.76 (1H, d, J = 11.9 Hz), 3.86 (1H, d, J = 11.9 Hz), 4.83 (1H, dd, J = 4.9, 11.5 Hz), 5.04 (1H, m), 5.39 (1H, dd, J = 5.1, 11.9 Hz), 646 (1H, s), 7.34-7.45 (2H, m), 7.86 (1H, dd, J = 1.3, 8.0 Hz), 8.08 (1H, dt, J = 2.0, 8.0 Hz), 8.64 (1H, dd, J = 1.2, 4.7 Hz), 8.68 (1H, dd, J = 1.5, 4.9 Hz), 9.00 (1H, d, J = 2.2 Hz) |

### Example 11

**Preparation Example 1 [Wettable powder]**

| | |
|---|---|
| Compound according to the present invention (Compound No. 82) | 30 wt% |
| Clay | 30 wt% |
| Diatomaceous earth | 35 wt% |
| Calcium lignin sulfonate | 4 wt% |
| Sodium laurylsulfate | 1 wt% |

The above ingredients were homogeneously mixed together, and the mixture was ground to prepare wettable powder.

**Preparation Example 2 [Dust]**

| | |
|---|---|
| Compound according to the present invention (Compound No. 82) | 2 wt% |
| Clay | 60 wt% |
| Talc | 37 wt% |
| Calcium stearate | 1 wt% |

The above ingredients were homogeneously mixed together to prepare dust.

**Preparation Example 3 [Emulsifiable concentrate]**

| | |
|---|---|
| Compound according to the present invention (Compound No. 82) | 20 wt% |
| N,N-Dimethylformamide | 20 wt% |
| Solvesso 150 (Exxon Mobil Corporation) | 50 wt% |
| Polyoxyethylene alkylaryl ether | 10 wt% |

The above ingredients were homogeneously mixed and dissolved to prepare emulsifiable concentrate.

**Preparation Example 4 [Granules]**

| | |
|---|---|
| Compound according to the present invention (Reference Compound No. 28) | 5 wt% |
| Bentonite | 40 wt% |
| Talc | 10 wt% |
| Clay | 43 wt% |
| Calcium lignin sulfonate | 2 wt% |

The above ingredients were homogeneously ground and homogeneously mixed together. Water was added to the mixture, followed by thorough kneading. Thereafter, the kneaded product was granulated and dried to prepare granules.

**Preparation Example 5 [Floables]**

| | |
|---|---|
| Compound according to the present invention (Reference Compound No. 28) | 25 wt% |
| POE polystyrylphenyl ether sulfate | 5 wt% |
| Propylene glycol | 6 wt% |
| Bentonite | 1 wt% |
| 1% aqueous xanthan gum solution PRONAL EX-300 | 3 wt% |
| (Toho Chemical Industry Co., Ltd.) ADDAC 827 | 0.05 wt% |
| (K.I. Chemical Industry Co., Ltd.) | 0.02 wt% |
| Water | To 100 wt% |

All the above ingredients except for the 1% aqueous xanthan gum solution and a suitable amount of water were premixed together, and the mixture was then ground by a wet grinding mill. Thereafter, the 1% aqueous xanthan gum solution and the remaining water were added to the ground product to prepare 100 wt% floables.

### Test Example 1: Pesticidal effect against Myzus persicae

Among the compounds of formula (I) produced by the conventional method described above, the compounds shown in Tables 1 to 14 and pyripyropene A were tested for pesticidal effect.

A leaf disk having a diameter of 2.8 cmφ was cut out from a cabbage grown in a pot and was placed in a 5.0 cm-Schale. Four adult aphids of Myzus persicae were released in the Schale. One day after the release of the adult aphids, the adult aphids were removed. The number of larvae at the first instar born in the leaf disk was adjusted to 10, and a test solution, which had been adjusted to a concentration of 20 ppm by the addition of a 50% aqueous acetone solution (0.05% Tween 20 added) was spread over the cabbage leaf disk. The cabbage leaf disk was then air dried. Thereafter, the Schale was lidded and was allowed to stand in a temperature-controlled room (light period 16 hr - dark period 8 hr) (25°C). Three days after the initiation of standing of the Schale, the larvae were observed for survival or death, and the death rate of larvae was calculated by the following equation. Death rate (%) = {number of dead larvae/(number of survived larvae + number of dead larvae)} x 100

As result, it was found that the death rate was not less than 80% for compounds of Nos. 1, 6, 8, 9, 10, 12, 14, 16, 18, 20, 23, 25, 28, 34, 35, 36, 37, 38, 39, 40, 44, 45, 49, 54, 56, 57, 61, 69, 76, 82, 85, 86, 88, 90, 91, 98, 103, 106, 107, 108, 109, 111, 125, 128, 133, 135, 137, 139, 142, 153, 160, 161, 162, 164, 167, 169, 170, 171, 172, 176, 180, 182, 183, 186, 187, 190, 196, 201, 205, 206, 207, 208, 209, 210, 211, 212, 213, 214, 215, 216, 217, 218, 219, 220, 221, 222, 223, 226, 227, 228, 229, 230, 231, 232, 233, 236, 237, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, and 274 and pyripyropene A.

### Test Example 2: Pesticidal effect against Myzus persicae

Among the compounds of formula (I) produced by the conventional method described above, the compounds shown in Tables 1 to 14 and pyripyropene A were tested for pesticidal effect.
A leaf disk having a diameter of 2.8 cmφ was cut out from a cabbage grown in a pot and was placed in a 5.0 cm-Schale. Four adult aphids of Myzus persicae were released in the Schale. One day after the release of the adult aphids, the adult aphids were removed. The number of larvae at the first instar born in the leaf disk was adjusted to 10, and a test solution, which had been adjusted to a concentration of 0.156 ppm by the addition of a 50% aqueous acetone solution (0.05% Tween 20 added) was spread over the cabbage leaf disk. The cabbage leaf disk was then air dried. Thereafter, the Schale was lidded and was allowed to stand in a temperature-controlled room (light period 16 hr - dark period 8 hr) (25°C). Three days after the initiation of standing, the larvae were observed for survival or death, and the death rate of larvae was calculated in the same manner as in Test Example 1.

As result, it was found that the death rate was not less than 80% for compounds of Nos. 12, 23, 28, 45, 54, 56, 76, 82, 85, 86, 90, 164, 201, 205, 206, 207, 212, 213, 217, 218, 219, 222, 227, 228, 229, 231, 232, 233, 237, 239, 240, 242, 246, 247, 249, 250, 252, 253, 256, 258, 261, 262, 264, 265, 266, 267, 269, 270, and 271.

### Test Example 3: Pesticidal effect against Plutella xylostella

A cabbage leaf disk having a diameter of 5 cm was placed in a plastic cup. Test compounds, which had been diluted to a predetermined concentration by the addition of a 50 % aqueous acetone solution(Tween 20, 0.05% added), were spreaded over the cabbage leaf disk by means of a spray gun, and the cabbage leaf disk was then air dried. Five larvae at the second instar of Plutella xylostella were released in the cup. The cup was then lidded, and the larvae were reared in the temperature-controlled room(25°C). Three days after the treatment, the larvae were observed for survival or death, and the death rate of the larvae was calculated in the same manner as in Test Example 1.

As a results, it was found that the death rate was not less than 80% for compounds of Nos. 76, 213, 218, 237 and 250 at a concentration of 500 ppm.

### Test Example 4: Pesticidal effect against Helicoverpa armigera

A cabbage leaf disk having a diameter of 2.8 cm was placed in a plastic cup. Test compounds, which had been diluted to a predetermined concentration by the addition of a 50 % aqueous acetone solution(Tween 20, 0.05% added), were spreaded over the cabbage leaf disk by means of a spray gun, and the cabbage leaf disk was then air dried. A larva at the third instar of Helicoverpa armigera was released in the cup. The cup was then lidded, and the larva was reared in the temperature-controlled room(25°C). Three days after the treatment, the larva was observed for survival or death. The test was repeated 5 times. Further, the death rate of the larvae were calculated in the same manner as in Test Example 1.

As a result, it was found that the death rate was not less than 80% for the compound of No. 219 at a concentration of 100 ppm.

### Test Example 5: Pesticidal effect against Trigonotylus caelestialium

A wheat seedling was immersed for 30 seconds in a solution, in which each test compound had been diluted to a predetermined concentration by the addition of a 50 % aqueous acetone solution(Tween 20, 0.05% added). The wheat seedling was air dried, and then placed in a glass cylinder. Further, two larvae at the second instar of Trigonotylus caelestialium were released in the glass cylinder. The glass cylinder was then lidded, and the larvae were reared in the temperature-controlled room(25°C). During the test, the wheat seedling was supplid with water from the bottom of the glass cylinder. Three days after the treatment, the larvae were observed for survival or death, and the death rate of the larvae were calculated in the same manner as in Test Example 1.

As a result, it was found that the death rate was not less than 80% for compound of Nos. 218 and 261 at a concentration of 100 ppm.

## Claims

1. Non-therapeutic use of a compound represented by formula (Ia), or an agriculturally and horticulturally acceptable salt thereof as a hemipteran pest control agent: 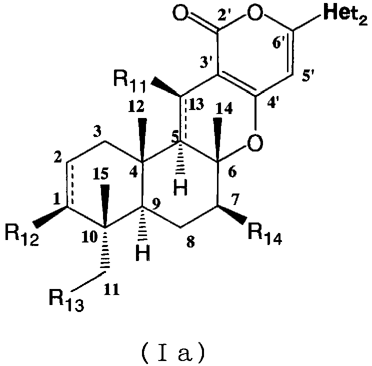 wherein
Het₂ represents 3-pyridyl,
R₁₁ represents hydroxyl,
R₁₂, R₁₃ and R₁₄ represents acetyloxy.

2. The use according to claim 1, wherein said hemipteran pest is selected from Aphidoidea, Coccoidea, or Aleyrodidae.

3. The use according to claim 1, wherein said hemipteran pest is at least one pest selected from the group consisting of Myzus persicae, Aphis gossypii, Aphis fabae, Aphis maidis (corn-leaf aphid), Acyrthosiphon pisum, Aulacorthum solani, Aphis craccivora, Macrosiphum euphorbiae, Macrosiphum avenae, Metopolophium dirhodum, Rhopalosiphum padi, Schizaphis graminum, Brevicoryne brassicae, Lipaphis erysimi, Aphis citricola, Rosy apple aphid, Eriosoma lanigerum, Toxoptera aurantii, Toxoptera citricidus, and Pseudococcus comstocki.

4. A compound represented by formula (Ib) or an agriculturally and horticulturally acceptable salt thereof: 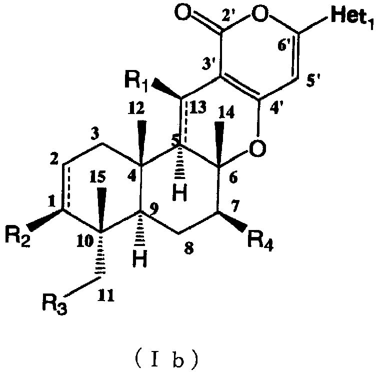 wherein
Het₁ represents 3-pyridyl,
R₁ represents hydroxyl,
R₂ and R₃ represent propionyloxy or
cyclic C₃₋₆ alkylcarbonyloxy optionally substituted by a halogen atom, cyclic C₃₋₆ alkyl or pyridyl, and
R₄ represents hydroxyl,
cyclic C₃₋₆ alkylcarbonyloxy optionally substituted by a halogen atom, cyclic C₃₋₆ alkyl or pyridyl,
benzoyloxy optionally substituted by a halogen atom, C₁₋₆ alkyl substituted by a halogen atom, cyano or nitro, or
saturated or unsaturated five- or six-membered heterocyclic carbonyloxy optionally substituted by a halogen atom, C_{1**-**4} alkyl, C₁₋₄ alkyloxy or trifluoromethyl,
wherein the saturated or unsaturated five-or six-membered heterocyclic carbonyloxy is selected from the group consisting of thienyl, furyl, pyrrolyl, imidazolyl, pyrazolyl, isothiazolyl, isoxazolyl, thiazolyl, oxazolyl, pyridyl, pyrimidinyl, pyrazinyl, pyridazinyl, tetrahydropyranyl, piperidinyl, piperazinyl, morpholinyl and mannosyl, and
wherein the alkyl is straight chain, branched chain, cyclic chain, or a combination thereof,
provided that
a compound wherein
Het₁ represents 3-pyridyl,
R₁ represents hydroxyl,
R₂ and R₃ represent propionyloxy, and
R₄ represents hydroxyl, and
a compound wherein
Het₁ represents 3-pyridyl,
R₁ represents hydroxyl,
R₂ and R₃ represent cyclopropylcarbonyloxy, and
R₄ represents hydroxyl, cyclopropylcarbonyloxy, or 2-cyanobenzoyloxy,
is excluded.

5. The compound according to claim 4, or an agriculturally and horticulturally acceptable salt thereof, wherein
R₂ and R₃ represent cyclic C₃₋₆ alkylcarbonyloxy_ optionally substituted by a halogen atom, cyclic C₃₋₆ alkyl or pyridyl, and
R₄ represents hydroxyl, cyclic C₃₋₆ alkylcarbonyloxy optionally substituted by a halogen atom, cyclic C₃₋₆ alkyl or pyridyl, or
benzoyloxy optionally substituted by a halogen atom, C₁₋₆ alkyl substituted by a halogen atom, cyano or nitro.

6. The compound according to claim 4, or an agriculturally and horticulturally acceptable salt thereof, wherein
R₂ and R₃ represent propionyloxy, and
R₄ represents cyclic C₃₋₆ alkylcarbonyloxy optionally substituted by a halogen atom, cyclic C₃₋₆ alkyl or pyridyl, or
saturated or unsaturated five- or six-membered heterocyclic carbonyloxy optionally substituted by a halogen atom, C₁₋₄ alkyl, C₁₋₄ alkyloxy or trifluoromethyl
wherein the saturated or unsaturated five-or six-membered heterocyclic carbonyloxy is selected from the group consisting of thienyl, furyl, pyrrolyl, imidazolyl, pyrazolyl, isothiazolyl, isoxazolyl, thiazolyl, oxazolyl, pyridyl, pyrimidinyl, pyrazinyl, pyridazinyl, tetrahydropyranyl, piperidinyl, piperazinyl, morpholinyl and mannosyl.

7. A composition for use as a pest control agent comprising the compound according to claim 4, or an agriculturally and horticulturally acceptable salt thereof as active ingredient and an agriculturally and horticulturally acceptable carrier.

8. A method for controlling a hemipteran pest, comprising applying an effective amount of a compound represented by formula (Ia) according to claim 1, or an agriculturally and horticulturally acceptable salt thereof to a plant or soil.

9. A method for controlling an pest, comprising applying an effective amount of a compound represented by formula (Ib) according to claim 4, or an agriculturally and horticulturally acceptable salt thereof to a plant or soil.

10. Non-therapeutic use of a compound represented by formula (Ib) according to claim 4, or an agriculturally and horticulturally acceptable salt thereof as a pest control agent.

## Patentansprüche

1. Nicht-therapeutische Verwendung einer durch die Formel (Ia) dargestellten Verbindung, oder eines landwirtschaftlich und gärtnerisch verträglichen Salzes davon als Hemipteran-Schädlingsbekämpfungsmittel: 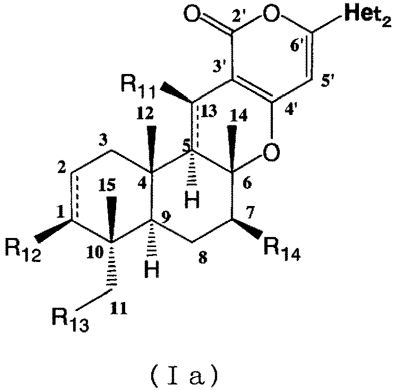 wobei
Het₂ 3-Pyridyl darstellt,
R₁₁ Hydroxyl darstellt,
R₁₂, R₁₃ und R₁₄ Acetyloxy darstellt.

2. Verwendung nach Anspruch 1, wobei der besagte Hemipteran-Schädling aus Aphidoidea, Coccoidea oder Aleyrodidae ausgewählt wird.

3. Verwendung nach Anspruch 1, wobei der besagte Hemipteran-Schädling zumindest ein Schädling ist, der aus der Gruppe ausgewählt wird, die sich aus Myzus persicae, Aphis gossypii, Aphis fabae, Aphis maidis (Maisblattlaus), Acyrthosiphon pisum, Aulacorthum solani, Aphis craccivora, Macrosiphum euphorbiae, Macrosiphum avenae, Metopolophium dirhodum, Rhopalosiphum padi, Schizaphis graminum, Brevicoryne brassicae, Lipaphis erysimi, Aphis citricola, mehlige Apfelblattlaus, Eriosoma lanigerum, Toxoptera aurantii, Toxoptera citricidus, und Pseudococcus comstocki zusammensetzt.

4. Verbindung, dargestellt durch die Formel (Ib), oder ein landwirtschaftlich und gärtnerisch verträgliches Salz davon: 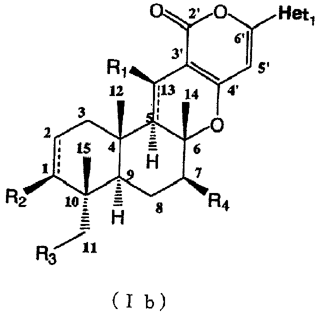 wobei
Het₁ 3-Pyridyl darstellt,
R₁ Hydroxyl darstellt,
R₂ und R₃ Propionyloxy oder
zyklisches C₃₋₆ Alkylcarbonyloxy darstellen, das optional durch ein Halogenatom, zyklisches C₃₋₆ Alkyl oder Pyridyl ersetzt wird, und
R₄ Hydroxyl,
zyklisches C₃₋₆ Alkylcarbonyloxy darstellt, das optional durch ein Halogenatom, zyklisches C₃₋₆ Alkyl oder Pyridyl ersetzt wird,
Benzoyloxy, das optional durch ein Halogenatom ersetzt wird, C₁₋₆ Alkyl, das durch ein Halogenatom ersetzt wird, Cyano oder Nitro, oder
gesättigtes oder ungesättigtes fünf- oder sechsgliedriges heterozyklisches Carbonyloxy, das optional durch ein Halogenatom, C₁₋₄ Alkyl, C₁₋₄ Alkyloxy oder Trifluoromethyl ersetzt wird,
wobei das gesättigte oder ungesättigte fünf- oder sechsgliedrige heterozyklische Carbonyloxy aus der Gruppe ausgewählt wird, die sich aus Thienyl, Furyl, Pyrrolyl, Imidazolyl, Pyrazolyl, Isothiazolyl, Isoxazolyl, Thiazolyl, Oxazolyl, Pyridyl, Pyrimidinyl, Pyrazinyl, Pyridazinyl, Tetrahydropyranyl, Piperidinyl, Piperazinyl, Morpholinyl und Mannosyl zusammensetzt, und
wobei das Alkyl eine gerade Kette, eine verzweigte Kette, eine zyklische Kette oder eine Kombination davon ist,
sofern
eine Verbindung, in der
Het₁ 3-Pyridyl darstellt,
R₁ Hydroxyl darstellt,
R₂ und R₃ Propionyloxy, und
R₄ Hydroxyl darstellt, und
eine Verbindung, in der
Het₁ 3-Pyridyl darstellt,
R₁ Hydroxyl darstellt,
R₂ und R₃ Cyclopropylcarbonyloxy darstellen, und
R₄ Hydroxyl, Cyclopropylcarbonyloxy, oder 2-Cyanobenzoyloxy darstellt,
ausgeschlossen ist.

5. Verbindung nach Anspruch 4, oder ein landwirtschaftlich und gärtnerisch verträgliches Salz davon, wobei
R₂ und R₃ zyklisches C₃₋₆ Alkylcarbonyloxy darstellen, das optional durch ein Halogenatom, zyklisches C₃₋₆ Alkyl oder Pyridyl ersetzt wird, und
R₄ Hydroxyl, zyklisches C₃₋₆ Alkylcarbonyloxy, das optional durch ein Halogenatom, zyklisches C₃₋₆ Alkyl oder Pyridyl ersetzt wird, oder
Benzoyloxy, das optional durch ein Halogenatom ersetzt wird, C₁₋₆ Alkyl, das durch ein Halogenatom, Cyano oder Nitro ersetzt wird, darstellt.

6. Verbindung nach Anspruch 4, oder ein landwirtschaftlich und gärtnerisch verträgliches Salz davon, wobei
R₂ und R₃ Propionyloxy darstellen, und
R₄ zyklisches C₃₋₆ Alkylcarbonyloxy darstellt, das optional durch ein Halogenatom, zyklisches C₃₋₆ Alkyl oder Pyridyl ersetzt wird,
oder
gesättigtes oder ungesättigtes fünf- oder sechsgliedriges heterozyklisches Carbonyloxy, darstellt, das optional durch ein Halogenatom, C₁₋₄ Alkyl, C₁₋₄ Alkyloxy oder Trifluoromethyl ersetzt wird
wobei das gesättigte oder ungesättigte fünf- oder sechsgliedrige heterozyklische Carbonyloxy aus der Gruppe ausgewählt wird, die sich aus Thienyl, Furyl, Pyrrolyl, Imidazolyl, Pyrazolyl, Isothiazolyl, Isoxazolyl, Thiazolyl, Oxazolyl, Pyridyl, Pyrimidinyl, Pyrazinyl, Pyridazinyl, Tetrahydropyranyl, Piperidinyl, Piperazinyl, Morpholinyl und Mannosyl zusammensetzt.

7. Zusammensetzung zur Verwendung als ein Schädlingsbekämpfungsmittel, umfassend die Verbindung nach Anspruch 4, oder ein landwirtschaftlich und gärtnerisch verträgliches Salz davon als aktiver Bestandteil und einen landwirtschaftlich und gärtnerisch verträglichen Träger.

8. Verfahren zur Kontrolle eines Hemipteran-Schädlings, umfassend das Aufbringen einer wirksamen Menge einer durch die Formel (Ia) dargestellten Verbindung nach Anspruch 1, oder eines landwirtschaftlich und gärtnerisch verträglichen Salzes davon auf eine Pflanze oder einen Boden.

9. Verfahren zur Kontrolle eines Schädlings, umfassend das Aufbringen einer wirksamen Menge einer durch die Formel (Ib) dargestellten Verbindung nach Anspruch 4, oder eines landwirtschaftlich und gärtnerisch verträglichen Salzes davon auf eine Pflanze oder einen Boden.

10. Nicht-therapeutische Verwendung einer durch die Formel (Ib) dargestellten Verbindung nach Anspruch 4, oder eines landwirtschaftlich und gärtnerisch verträglichen Salzes davon als ein Schädlingsbekämpfungsmittel.

## Revendications

1. Utilisation non-thérapeutique d'un composé représenté par la formule (Ia), ou d'un sel de celui-ci acceptable sur les plans agricole et horticole en tant qu'agent de lutte contre les insectes nuisibles hémiptères : 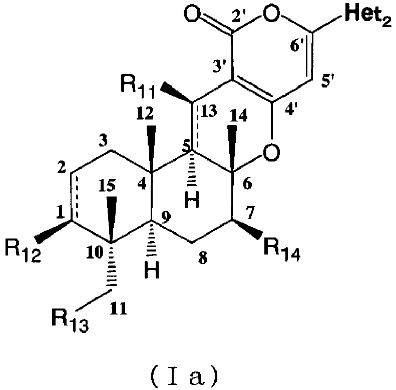 dans laquelle
Het₂ représente un groupe 3-pyridyle,
R₁₁ représente un groupe hydroxyle,
R₁₂, R₁₃ et R₁₄ représentent un groupe acétyloxy.

2. Utilisation selon la revendication 1, dans laquelle ledit insecte nuisible est sélectionné parmi Aphidoidea, Coccoidea, ou Aleyrodidae.

3. Utilisation selon la revendication 1, dans laquelle ledit insecte nuisible hémiptère est au moins un insecte nuisible sélectionné dans le groupe constitué de Myzus persicae, Aphis gossypii, Aphis fabae, Aphis maidis (puceron du maïs), Acyrthosiphon pisum, Aulacorthum solani, Aphis craccivora, Macrosiphum euphorbiae, Macrosiphum avenae, Metopolophium dirhodum, Rhopalosiphum padi, Schizaphis graminum, Brevicoryne brassicae, Lipaphis erysimi, Aphis citricola, puceron rose du pommier, Eriosoma lanigerum, Toxoptera aurantii, Toxoptera citricidus, et Pseudococcus comstocki.

4. Composé représenté par la formule (Ib) ou un sel de celui-ci acceptable sur les plans agricole et horticole : 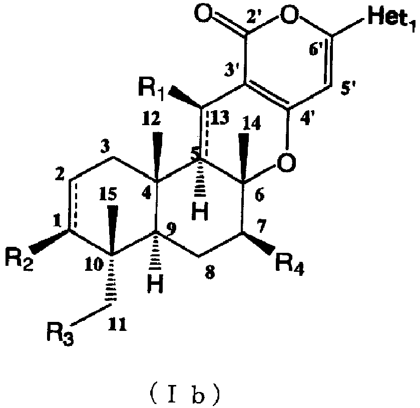 dans laquelle
Het₁ représente un groupe 3-pyridyle,
R₁ représente un groupe hydroxyle,
R₂ et R₃ représentent un groupe propionyloxy ou
un groupe C₃₋₆ alkylcarbonyloxy cyclique facultativement substitué par un atome d'halogène, un groupe C₃₋₆ alkyle cyclique ou pyridyle, et
R₄ représente un groupe hydroxyle,
un groupe C₃₋₆ alkylcarbonyloxy cyclique facultativement substitué par un atome d'halogène, un groupe C₃₋₆ alkyle cyclique ou pyridyle,
un groupe benzoyloxy facultativement substitué par un atome d'halogène, un groupe C₁₋₆ alkyle substitué par un atome d'halogène, un groupe cyano ou nitro, ou
un groupe carbonyloxy hétérocyclique à cinq ou six chaînons saturé ou insaturé facultativement substitué par un atome d'halogène, un groupe C₁₋₄ alkyle, un groupe C₁₋₄ alkyloxy ou un groupe trifluoroméhtyle,
dans lequel le groupe carbonyloxy hétérocyclique à cinq ou six chaînons saturé ou insaturé est sélectionné dans le groupe constitué des groupes thiényle, furyle, pyrrolyle, imidazolyle, pyrazolyle, isothiazolyle, isoxazolyle, thiazolyle, oxazolyle, pyridyle, pyrimidinyle, pyrazinyle, pyridazinyle, tétrahydropyranyle, pipéridinyle, pipérazinyle, morpholinyle et mannosyle, et
dans lequel l'alkyle est à chaîne linéaire, à chaîne ramifiée, à chaîne cyclique, ou une combinaison de ceux-ci,
dans la mesure où
un composé dans lequel
Het₁ représente un groupe 3-pyridyle,
R₁ représente un groupe hydroxyle,
R₂ et R₃ représentent un groupe propionyloxy, et
R₄ représente un groupe hydroxyle, et
un composé dans lequel
Het₁ représente un groupe 3-pyridyle,
R₁ représente un groupe hydroxyle,
R₂ et R₃ représentent un groupe cyclopropylcarbonyloxy, et
R₄ représente un groupe hydroxyle, cyclopropylcarbonyloxy, ou 2-cyanobenzoyloxy,
est exclu.

5. Composé selon la revendication 4, ou un sel de celui-ci acceptable sur les plans agricole et horticole, dans lequel
R₂ et R₃ représentent un groupe C₃₋₆ alkylcarbonyloxy cyclique facultativement substitué par un atome d'halogène, un groupe C₃₋₆ alkyle cyclique ou pyridyle, et
R₄ représente un groupe hydroxyle, C₃₋₆ alkylcarbonyloxy cyclique facultativement substitué par un atome d'halogène, un groupe C₃₋₆ alkyle cyclique ou pyridyle, ou
un groupe benzoyloxy facultativement substitué par un atome d'halogène, un groupe C₁₋₆ alkyle substitué par un atome d'halogène, un groupe cyano ou nitro.

6. Composé selon la revendication 4, ou un sel de celui-ci acceptable sur les plans agricole et horticole, dans lequel
R₂ et R₃ représentent un groupe propionyloxy, et
R₄ représente un groupe C₃₋₆ alkylcarbonyloxy cyclique facultativement substitué par un atome d'halogène, un groupe C₃₋₆ alkyle cyclique ou pyridyle, ou
un groupe carbonyloxy hétérocyclique à cinq ou six chaînons saturé ou insaturé facultativement substitué par un atome d'halogène, un groupe C₁₋₄ alkyle, un groupe C₁₋₄ alkyloxy ou un groupe trifluoroméhtyle,
dans lequel le groupe carbonyloxy hétérocyclique à cinq ou six chaînons saturé ou insaturé est sélectionné dans le groupe constitué des groupes thiényle, furyle, pyrrolyle, imidazolyle, pyrazolyle, isothiazolyle, isoxazolyle, thiazolyle, oxazolyle, pyridyle, pyrimidinyle, pyrazinyle, pyridazinyle, tétrahydropyranyle, pipéridinyle, pipérazinyle, morpholinyle et mannosyle.

7. Composition pour une utilisation en tant qu'agent de lutte contre les insectes nuisibles comprenant le composé selon la revendication 4, ou un sel de celui-ci acceptable sur les plans agricole et horticole en tant que principe actif et un vecteur acceptable sur les plans agricole et horticole.

8. Méthode de lutte contre un insecte nuisible hémiptère, comprenant l'application d'une quantité efficace d'un composé représenté par la formule (Ia) selon la revendication 1, ou d'un sel de celui-ci acceptable sur les plans agricole et horticole à une plante ou un sol.

9. Méthode de lutte contre un insecte nuisible, comprenant l'application d'une quantité efficace d'un composé représenté par la formule (Ib) selon la revendication 4, ou d'un sel de celui-ci acceptable sur les plans agricole et horticole à une plante ou un sol.

10. Utilisation non-thérapeutique d'un composé représenté par la formule (Ib) selon la revendication 4, ou d'un sel de celui-ci acceptable sur les plans agricole et horticole en tant qu'agent de lutte contre les insectes nuisibles.
